# EUROPEAN PATENT SPECIFICATION

(11) **EP 2 753 629 B1**
(45) Date of publication and mention of the grant of the patent: **19.10.2016**
(21) Application number: 12829439.4
(22) Date of filing: 06.09.2012
(51) Int. Cl.: C07H 1/08, C12Q 1/68, C12N 15/10

(54) **METHODS FOR DETECTING LYME DISEASE**
VERFAHREN FÜR DEN NACHWEIS VON LYME-KRANKHEIT
PROCÉDÉ POUR DÉTECTER LA MALADIE DE LYME

(30) Priority: 06.09.2011 US 201161531471 P
(43) Date of publication of application: 16.07.2014
(73) Proprietor: Ibis Biosciences, Inc., Carlsbad, CA 92008 (US)
(72) Inventor: ESHOO, Mark W., San Diego, California 92130 (US); CROWDER, Christopher, San Marcos, California 92078 (US)
(74) Representative: Chapman, Desmond Mark
(86) International application number: PCT/US2012/053909
(87) International publication number: WO 2013/036603

(56) References cited:
- US-A1- 2003 054 338
- US-A1- 2006 078 923
- US-A1- 2010 190 240
- D. LIVERIS ET AL: "Improving the Yield of Blood Cultures from Patients with Early Lyme Disease", JOURNAL OF CLINICAL MICROBIOLOGY, vol. 49, no. 6, 13 April 2011 (2011-04-13) , pages 2166-2168, XP055181851, ISSN: 0095-1137, DOI: 10.1128/JCM.00350-11
- VAN DAM ALJE P: "Molecular diagnosis of Borrelia bacteria for the diagnosis of Lyme disease.", EXPERT OPINION ON MEDICAL DIAGNOSTICS MAR 2011, vol. 5, no. 2, March 2011 (2011-03), pages 135-149, XP009183653, ISSN: 1753-0059
- BABADY N E; PATEL R; SLOAN L M; VETTER E A; SPURBECK D K; BINNICKER M J: "Positivity Rate for Detection of Borrelia burgdorferi in Blood, Cerebrospinal Fluid, Synovial Fluid and Tissue by Real-Time PCR", ABSTRACTS OF THE GENERAL MEETING OF THE AMERICAN SOCIETY FOR MICROBIOLOGY., vol. 108, 1 June 2008 (2008-06-01), XP055181911, US ISSN: 1060-2011
- LIVERIS D ET AL: "Quantitation of cell-associated borrelial DNA in the blood of Lyme disease patients with erythema migrans", EUROPEAN JOURNAL OF CLINICAL MICROBIOLOGY & INFECTIOUS DISEASES, SPRINGER, BERLIN, DE, vol. 31, no. 5, 16 August 2011 (2011-08-16), pages 791-795, XP035039694, ISSN: 1435-4373, DOI: 10.1007/S10096-011-1376-X
- FOMENKO N V ET AL: "Diversity of Borrelia burgdorferi sensu lato in natural foci of Novosibirsk region", INTERNATIONAL JOURNAL OF MEDICAL MICROBIOLOGY, URBAN UND FISCHER, DE, vol. 298, 1 September 2008 (2008-09-01), pages 139-148, XP023520152, ISSN: 1438-4221, DOI: 10.1016/J.IJMM.2007.11.008 [retrieved on 2008-01-30]
- E. POSTNIKOVA ET AL: "Identification of Bacterial Plant Pathogens Using Multilocus Polymerase Chain Reaction/Electrospray Ionization-Mass Spectrometry", PHYTOPATHOLOGY, vol. 98, no. 11, 1 November 2008 (2008-11-01), pages 1156-1164, XP055182137, ISSN: 0031-949X, DOI: 10.1094/PHYTO-98-11-1156
- HOFSTADLER S A: "High throughput characterization of amplified nucleic acids by ESI-TOF mass spectrometry: Applications in pathogen detection and characterization", MOLECULAR & CELLULAR PROTEOMICS, vol. 6, no. 8, Suppl. S, August 2007 (2007-08), page 66, XP055182186, & 8TH INTERNATIONAL SYMPOSIUM ON MASS SPECTROMETRY IN THE HEALTH AND LIFE SCIENCES; SAN FRANCISCO, CA, USA; AUGUST 18 -23, 2007 ISSN: 1535-9476
- MARK W. ESHOO ET AL: "Direct Molecular Detection and Genotyping of Borrelia burgdorferi from Whole Blood of Patients with Early Lyme Disease", PLOS ONE, vol. 7, no. 5, 8 May 2012 (2012-05-08), page e36825, XP055182231, DOI: 10.1371/journal.pone.0036825

## Description

### FIELD OF THE INVENTION

The present invention provides methods of detecting Lyme disease comprising whole blood nucleic acid extraction methods, as well as nested isothermal amplification methods. In certain embodiments, these methods are applied to detecting Lyme disease in patients without classic erythema migrans skin lesions.

### BACKGROUND

The diagnosis of acute infectious diseases is problematic when the pathogens are not in abundance, not easily cultured or the antibody response to them is delayed. Lyme disease is such an example. Infection by the causative agent, *Borrelia burgdorferi^{1,2}*, is often difficult to diagnose because of clinical variability, including variations of the erythema migrans (EM) skin lesion and the lack of high performing diagnostic tests. A strategy that results in early unambiguous diagnosis of the infection will favorably impact management of the patient.

There are currently clinical limitations to early diagnosis. The best clinical marker of acute infection, the skin lesion erythema migrans (EM), is absent in at least 30% of the cases.³ Even when present, the EM is often not the classic bull's-eye lesion but rather an uncharacteristic variant as reported in 25-30% of cases.^{4,5} This may require referral to a specialist or prompt further diagnostic testing, thereby delaying the start of antibiotic therapy at the time for the best chance of cure without sequelae.

These clinical hurdles to diagnosis are compounded by the limitations of current laboratory tests. The most commonly used assays are serologic. However, these assays have been hampered by the biologically delayed antibody response (often 3 weeks or more to reach detectable levels). They are further limited because a *single* positive test is an indirect measure of exposure and cannot distinguish active from past infection. A direct *Borrelia* molecular test such as PCR can have high specificity but typically the tests have lacked sensitivity when applied to blood. In the past, PCR assays for Lyme disease did not have the benefit of current advances in sample preparation.⁶ and enhancement methods.^{7,8} such as *Borrelia*-targeted DNA enrichment (akin to a "molecular culture") that may allow detection of a low number of nucleic acid copies in the blood. In addition, the optimal blood volume and component of the sample, such as whole blood versus serum, may have contributed to prior limitations of PCR based diagnostics.

### SUMMARY OF THE INVENTION

The present invention provides methods of detecting Lyme disease comprising whole blood nucleic acid extraction methods, as well as nested isothermal amplification methods. In certain embodiments, these methods are applied to detecting Lyme disease in patients without classic erythema migrans skin lesions.

In particular, the present invention provides a method of detecting Lyme disease, comprising: (1) a method of nucleic acid extraction comprising: (a) contacting a sample of whole blood with beads, a proteinase, and an anionic surfactant to generate a treated sample; (b) homogenizing said treated sample to generate a cell lysate; (c) centrifuging said cell lysate comprising a supernatant; (d) separating said supernatant from said cell lysate; (e) adding magnetic particles and lysis buffer to said supernatant to generate a magnetic-particle sample, wherein said magnetic particles are configured to bind nucleic acid molecules; (f) washing said magnetic-particle sample with a wash buffer; (g) treating said magnetic-particle sample in order to generate a dried magnetic bead sample; and (h) treating said dried magnetic bead sample with an elution buffer such that a purified nucleic acid sample is generated that comprises purified nucleic acid; and (2) a nested isothermal amplification method comprising: (a) contacting the purified nucleic acid sample with a buffer, dNTPs, and a plurality of nested PCR primer pairs configured to amplify at least part of at least one nucleic acid sequence from *Borrelia burgdorferi*; (b) incubating said purified nucleic acid sample with a DNA polymerase under isothermal conditions such that amplified nucleic acid is generated; (c) inactivating said DNA polymerase; and (d) subjecting said amplified nucleic acid to mass spectrometry to identify *Borrelia burgdorferi* in the sample.

In some embodiments, the mass spectrometry comprises electrospray ionization mass spectrometry and base composition analysis. In certain embodiments, the DNA polymerase comprises BstE DNA polymerase. In additional embodiments, the incubating is conducted at about 56 degrees Celsius. In further embodiments, inactivating the DNA polymerase comprises heating the sample to at least about 80 degrees Celsius. In other embodiments, the plurality of nested PCR primer pairs comprises at least 10 primer pairs (e.g., 10 ... 15 ... 19, etc). In particular embodiments, the plurality of nested PCR primer pairs comprises at least 20 primer pairs (e.g., 20 ... 25 ... 35 ... 45 ... etc.). In further embodiments, the at least one nucleic acid sequence from *Borrelia burgdorferi* comprises at least five nucleic acid sequences from *Borrelia burgdorferi.* In certain embodiments, the methods further comprise a step after (2)(c) but before (2)(d) of further amplifying the amplified nucleic acid without any purification of the amplified nucleic acid.

### DESCRIPTION OF THE FIGURES

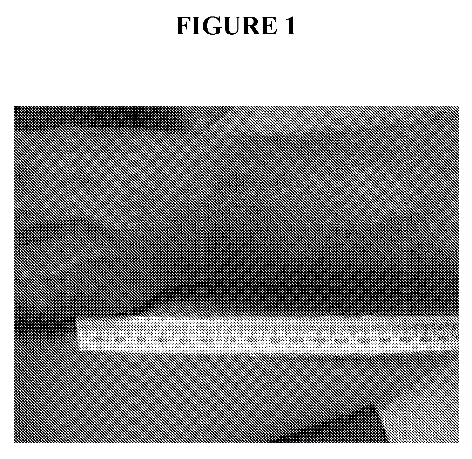

Figure 1 shows an atypical EM lesion from a patient who was PCR positive and seronegative with a negative ELISA after eight days of illness. Repeat serology at the end of therapy 3 weeks later showed a positive ELISA, positive IgM western blot and negative IgG western blot.

### DETAILED DESCRIPTION

The present invention provides methods of detecting Lyme disease comprising whole blood nucleic acid extraction methods, as well as nested isothermal amplification methods. In certain embodiments, these methods are applied to detecting Lyme disease in patients without classic erythema migrans skin lesions.

The methods, kits, and compositions disclosed herein are useful with any target nucleic acid sequence and are not limited to any particular target sequence (e.g., not limited to nucleic acid sequences from *B. burgdorferi*). The discussion below is focused on detecting sequence from *B. burgdorferi.* Such target sequences are exemplary and are not intended to limit the scope of the present description. Other target sequences (e.g., from infections disease) are well known in the art. Also well know in the art are primers, including nested prior sets, that are useful in the present description.

Lyme disease is representative of an infectious disease where early diagnosis is imperative to avoid sequelae. However diagnosis is often difficult because the clinical manifestations, including the rash, are variable and the pathogens are often not in abundance, not easily cultured, and the antibody response to them is delayed. In the past PCR for *B. burgdorferi* in blood had low sensitivity. This may be related to low copy number, insufficient volume of blood or targeting the wrong component of blood

The present invention overcomes some of these obstacles by combining a pre-PCR nucleic acid enrichment with sensitive PCR detection from nucleic acid extraction from 1.25 ml of whole blood from patients with skin lesions and early Lyme disease. With this strategy, PCR positivity was found in 14 of 29 (p=0·0001) endemic area subjects with typical erythema migrans (EM) or a variant, often before seroconversion was positive. The enrichment technique increased the yield from 2 to 14. None of 44 control subjects were positive. A serendipitous and unexpected finding of clinical importance was the observation that 8 of 14 (57%) of PCR positive subjects had an atypical EM.

The invention provides improved early diagnosis of Lyme disease by combination of a pre-PCR *Borrelia* DNA enhancement, sensitive PCR, and targeting sufficient volumes of whole blood. The surprise finding of non-classic EM lesions in the majority of microbiologically proven Lyme disease cases serves as alert to clinicians evaluating patients with endemic area exposure.

In certain embodiments, the PCR generated amplicons are detected by mass spectrometry methods using bioagent identifying amplicons. In some embodiments, primers are selected to hybridize to conserved sequence regions of nucleic acids derived from a bioagent and which flank variable sequence regions to yield a bioagent identifying amplicon which can be amplified and which is amenable to base composition analysis. In some embodiments, the corresponding base composition of one or more different amplicons is queried against a database of base compositions indexed to bioagents and to the primer pair used to generate the amplicon. A match of the measured base composition to a database entry base composition associates the sample bioagent to an indexed bioagent in the database. Thus, the identity of the unknown bioagent is determined. No prior knowledge of the unknown bioagent is necessary to make an identification. In some instances, the measured base composition associates with more than one database entry base composition. Thus, a second/subsequent primer pair is generally used to generate an amplicon, and its measured base composition is similarly compared to the database to determine its identity in triangulation identification. Furthermore, the methods of the invention can be applied to rapid parallel multiplex analyses, the results of which can be employed in a triangulation identification strategy. Thus, in some embodiments, the present invention provides rapid throughput and does not require nucleic acid sequencing or knowledge of the linear sequences of nucleobases of the amplified target sequence for bioagent detection and identification.

Methods of employing base compositions, databases containing base composition entries, and triangulation using primers, are described in the following patents, patent applications and scientific publications: US patent numbers 7,108,974; 7,217,510; 7,226,739; 7,255,992; 7,312,036; 7,339,051; US patent publication numbers 2003/0027135; 2003/0167133; 2003/0167134; 2003/0175695; 2003/0175696; 2003/0175697; 2003/0187588; 2003/0187593; 2003/0190605; 2003/0225529; 2003/0228571; 2004/0110169; 2004/0117129; 2004/0121309; 2004/0121310; 2004/0121311; 2004/0121312; 2004/0121313; 2004/0121314; 2004/0121315; 2004/0121329; 2004/0121335; 2004/0121340; 2004/0122598; 2004/0122857; 2004/0161770; 2004/0185438; 2004/0202997; 2004/0209260; 2004/0219517; 2004/0253583; 2004/0253619; 2005/0027459; 2005/0123952; 2005/0130196 2005/0142581; 2005/0164215; 2005/0266397; 2005/0270191; 2006/0014154; 2006/0121520; 2006/0205040; 2006/0240412; 2006/0259249; 2006/0275749; 2006/0275788; 2007/0087336; 2007/0087337; 2007/0087338 2007/0087339; 2007/0087340; 2007/0087341; 2007/0184434; 2007/0218467; 2007/0218467; 2007/0218489; 2007/0224614; 2007/0238116; 2007/0243544; 2007/0248969; WO2002/070664; WO2003/001976; WO2003/100035; WO2004/009849; WO2004/052175; WO2004/053076; WO2004/053141; WO2004/053164; WO2004/060278; WO2004/093644; WO 2004/101809; WO2004/111187; WO2005/023083; WO2005/023986; WO2005/024046; WO2005/033271; WO2005/036369; WO2005/086634; WO2005/089128; WO2005/091971; WO2005/092059; WO2005/094421; WO2005/098047; WO2005/116263; WO2005/117270; WO2006/019784; WO2006/034294; WO2006/071241; WO2006/094238; WO2006/116127; WO2006/135400; WO2007/014045; WO2007/047778; WO2007/086904; WO2007/100397; and WO2007/118222.

Exemplary base-count related methods and other aspects of use in the methods of the invention are also described in, for example, Ecker et al., Ibis T5000: a universal biosensor approach for microbiology. Nat Rev Microbiol. 2008 Jun 3.; Ecker *et al.,* The Microbial Rosetta Stone Database: A compilation of global and emerging infectious microorganisms and bioterrorist threat agents.; Ecker *et al.,* The Ibis T5000 Universal Biosensor: An Automated Platform for Pathogen Identification and Strain Typing.; Ecker *et al.,* The Microbial Rosetta Stone Database: A common structure for microbial biosecurity threat agents.; Ecker et al., Identification of Acinetobacter species and genotyping of Acinetobacter baumannii by multilocus PCR and mass spectrometry. J Clin Microbiol. 2006 Aug;44(8):2921-32.; Ecker et al., Rapid identification and strain-typing of respiratory pathogens for epidemic surveillance. Proc Natl Acad Sci U S A. 2005 May 31;102(22):8012-7. Epub 2005 May 23.; Wortmann et al., Genotypic evolution of Acinetobacter baumannii Strains in an outbreak associated with war trauma, Infect Control Hosp Epidemiol. 2008 Jun;29(6):553-555.; Hannis et al., High-resolution genotyping of Campylobacter species by use of PCR and high-throughput mass spectrometry. J Clin Microbiol. 2008 Apr;46(4): 1220-5.; Blyn et al., Rapid detection and molecular serotyping of adenovirus by use of PCR followed by electrospray ionization mass spectrometry. J Clin Microbiol. 2008 Feb;46(2):644-51.; Eshoo et al., Direct broad-range detection of alphaviruses in mosquito extracts, Virology. 2007 Nov 25;368(2):286-95.; Sampath et al., Global surveillance of emerging Influenza virus genotypes by mass spectrometry. PLoS ONE. 2007 May 30;2(5):e489.; Sampath et al., Rapid identification of emerging infectious agents using PCR and electrospray ionization mass spectrometry. Ann N Y Acad Sci. 2007 Apr;1102: 109-20.; Hujer et al., Analysis of antibiotic resistance genes in multidrug-resistant Acinetobacter sp. isolates from military and civilian patients treated at the Walter Reed Army Medical Center. Antimicrob Agents Chemother. 2006 Dec;50(12):4114-23.; Hall et al., Base composition analysis of human mitochondrial DNA using electrospray ionization mass spectrometry: a novel tool for the identification and differentiation of humans. Anal Biochem. 2005 Sep 1;344(1):53-69.; Sampath et al., Rapid identification of emerging pathogens: coronavirus. Emerg Infect Dis. 2005 Mar;11(3):373-9.; Jiang Y, Hofstadler SA. A highly efficient and automated method of purifying and desalting PCR products for analysis by electrospray ionization mass spectrometry; Jiang et al., Mitochondrial DNA mutation detection by electrospray mass spectrometry; Russell et al., Transmission dynamics and prospective environmental sampling of adenovirus in a military recruit setting; Hofstadler et al., Detection of microbial agents using broad-range PCR with detection by mass spectrometry: The TIGER concept. Chapter in; Hofstadler et al., Selective ion filtering by digital thresholding: A method to unwind complex ESI-mass spectra and eliminate signals from low molecular weight chemical noise.; Hofstadler et al., TIGER: The Universal Biosensor.; Van Ert et al., Mass spectrometry provides accurate characterization of two genetic marker types in Bacillus anthracis.; Sampath et al., Forum on Microbial Threats: Learning from SARS: Preparing for the Next Disease Outbreak -- Workshop Summary (ed. Knobler SE, Mahmoud A, Lemon S.) The National Academies Press, Washington, D.C. 2004. 181-185.

### EXAMPLES

### EXAMPLE 1

### Early Lyme patient specimens.

The current Example is part of a larger, ongoing longitudinal cohort study of early Lyme disease being conducted in a suburban community of a medium-sized, Northeast city since the summer of 2008. Adult patients with early, untreated Lyme disease are referred to a primary care physician with infectious disease training (JA) and provide written consent to participate.

Eligible patients are required to be treatment naïve, to have a documented rash diagnosed as EM at time of enrollment, and to have evidence of systemic infection; typically manifest as dissemination of the primary EM lesion or concurrent onset of new flu-like or other symptoms. Patients with a prior history of Lyme disease or symptom duration of their current illness of longer than 3 months are excluded. ²⁰. Forty four negative control specimens were obtained from Biomed Supply Inc. (Carlsbad, CA). Specimens were collected at a donation site in Pennsylvania from healthy donors screened by Biomed Supply Inc. A paired 7mL tube of EDTA treated whole blood and 5-12mL of serum was provided for each control patient.

### Serological and other analyses.

At the initial, pre-treatment study visit, self-report demographics, medical history information, complete blood counts (CBC), complete metabolic panels (CMP) and 2-tier antibody testing for *B. burgdorferi* were performed as part of the patient clinical evaluation. All serologic testing for both the patient specimens and the negative controls was performed through the same commercial laboratory, and results were interpreted according to CDC criteria ¹¹. Based on these criteria, approximate illness duration was documented and included in determining serostatus; patients with less than 4 weeks illness duration were positive based on either IgM or IgG positivity on western blot analysis, whereas patients with greater than 4 weeks illness duration were required to be IgG positive. Photos of EM rashes were reviewed by a panel of experienced clinicians without knowledge of serologic or PCR status. Skin lesions with classic bull's eye appearance with central clearing and peripheral erythema were classified as classic EM lesions. EM with uniform red or red-blue appearance which lacked central clearing were classified as non-classic EM lesions.

### DNA extraction from blood specimens:

A combination of bead-beating and magnetic bead isolation was used to extract nucleic acids from 1.25mL of whole EDTA blood. The blood was mixed in 2.0 mL screw-cap tubes (Sarstedt, Newton NC) filled with 1.35 g of 0.1-mm yttriastabilized zirconium oxide beads (Glen Mills, Clifton, NJ), 25 µL proteinase K solution (Qiagen, Valencia, CA), 142 µL of 20% SDS solution (Ambion, Austin, TX) and 1uL of DNA extraction control (Abbott Molecular, Des Plains, IL). The mixture was then homogenized in a Precellys 24 tissue homogenizer (Bioamerica Inc., Miami, FL) at 6,200 rpm for 3 sets of 90 sec with 5 sec between sets. The homogenized lysates were then incubated at 56°C for 15 min and then centrifuged for 3 min at 16,000 g in a bench top microcentrifuge. To isolate nucleic acids 1 mL of the supernatant was transferred to a 24-well deep-well Kingfisher plate (Thermo Scientific, Waltham, MA) along with 1.1 mL of Abbott lysis buffer (Abbott Molecular), and 160 µL of magnetic particles (Abbott Molecular). The specimens were incubated for 16.5 minutes in the lysis buffer at 56°C. Specimens were then washed once in Wash buffer I (Abbott Molecular), and three times in Wash buffer II (Abbott Molecular), with 1 min incubation for each wash step. The magnetic beads were then dried for 3 min at 65°C, and nucleic acids were eluted into 250 µL of elution buffer (Abbott Molecular), by incubating the magnetic particles at 65°C for 3 min.

### Nested isothermal amplification of B. burgdorferi target DNA.

To increase sensitivity of *B. burgdorferi* detection by PCR/ESI-MS the seven target regions (eight primer pairs) of the previously described *Borrelia* genotyping assay²¹ were enriched by a nested isothermal amplification. For each of the seven target regions were amplified using 50 oligonucleotide primers: 25 upstream and 25 downstream to the DNA region of interest (Table 1).

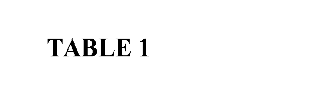

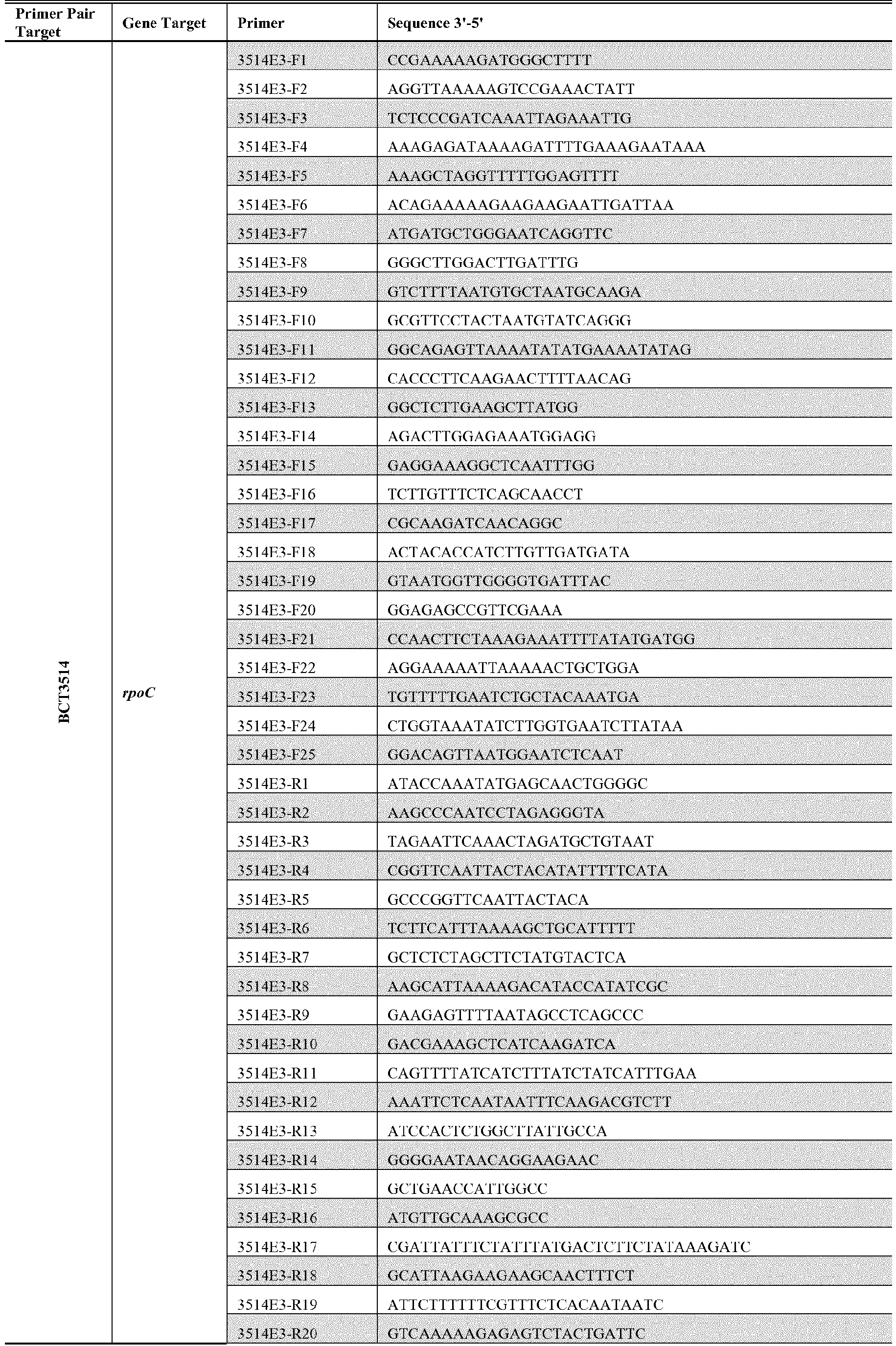

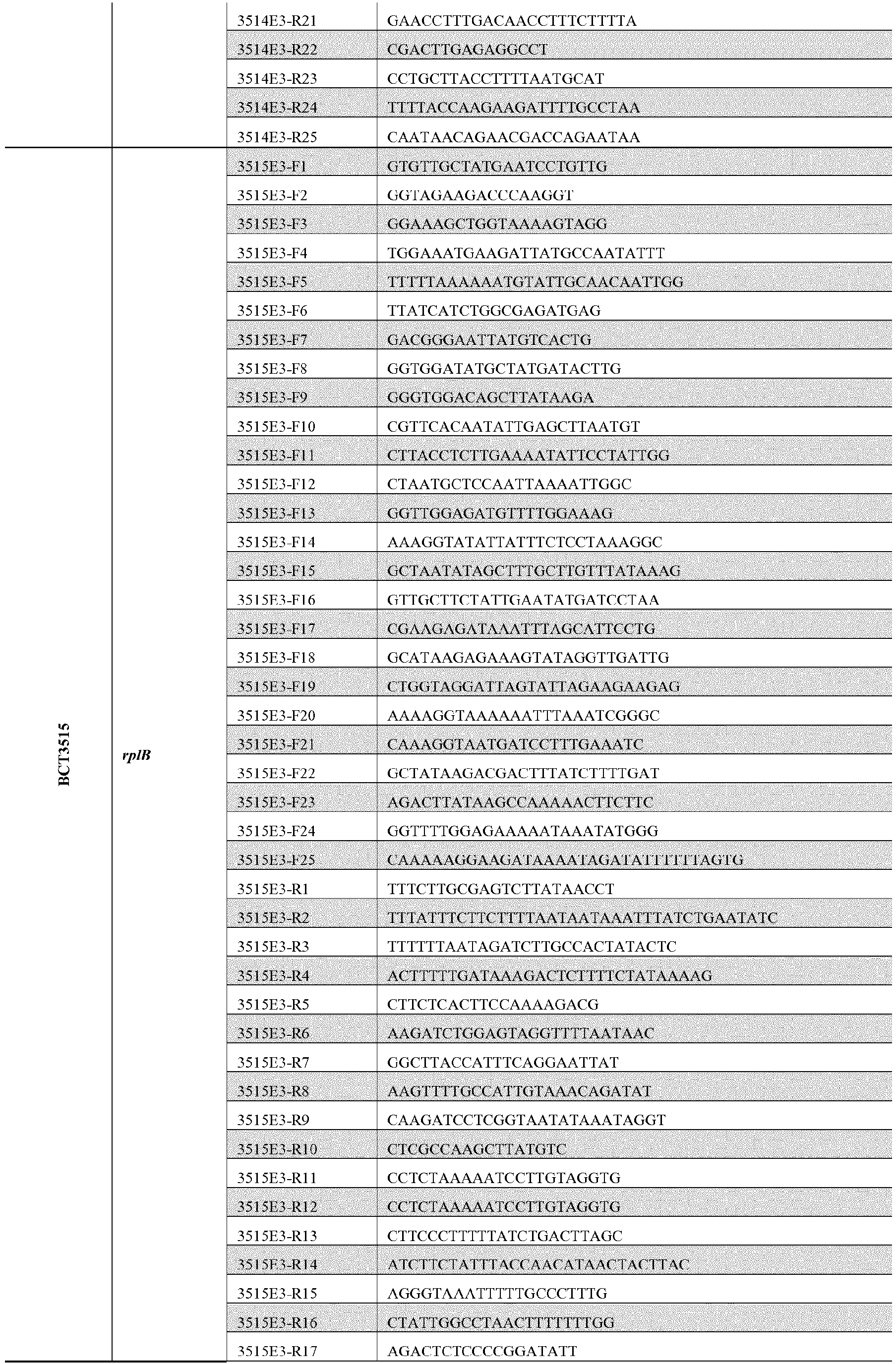

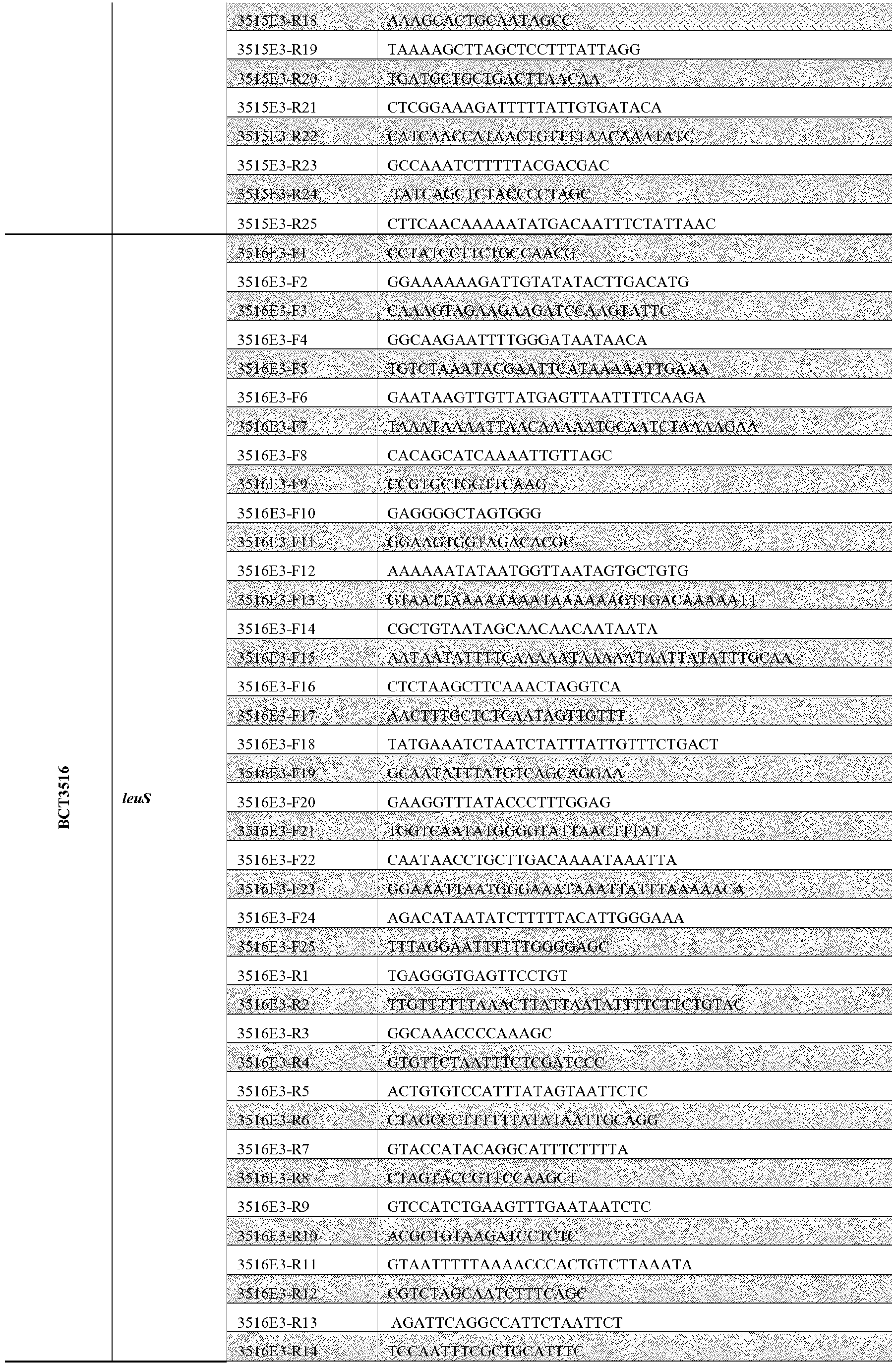

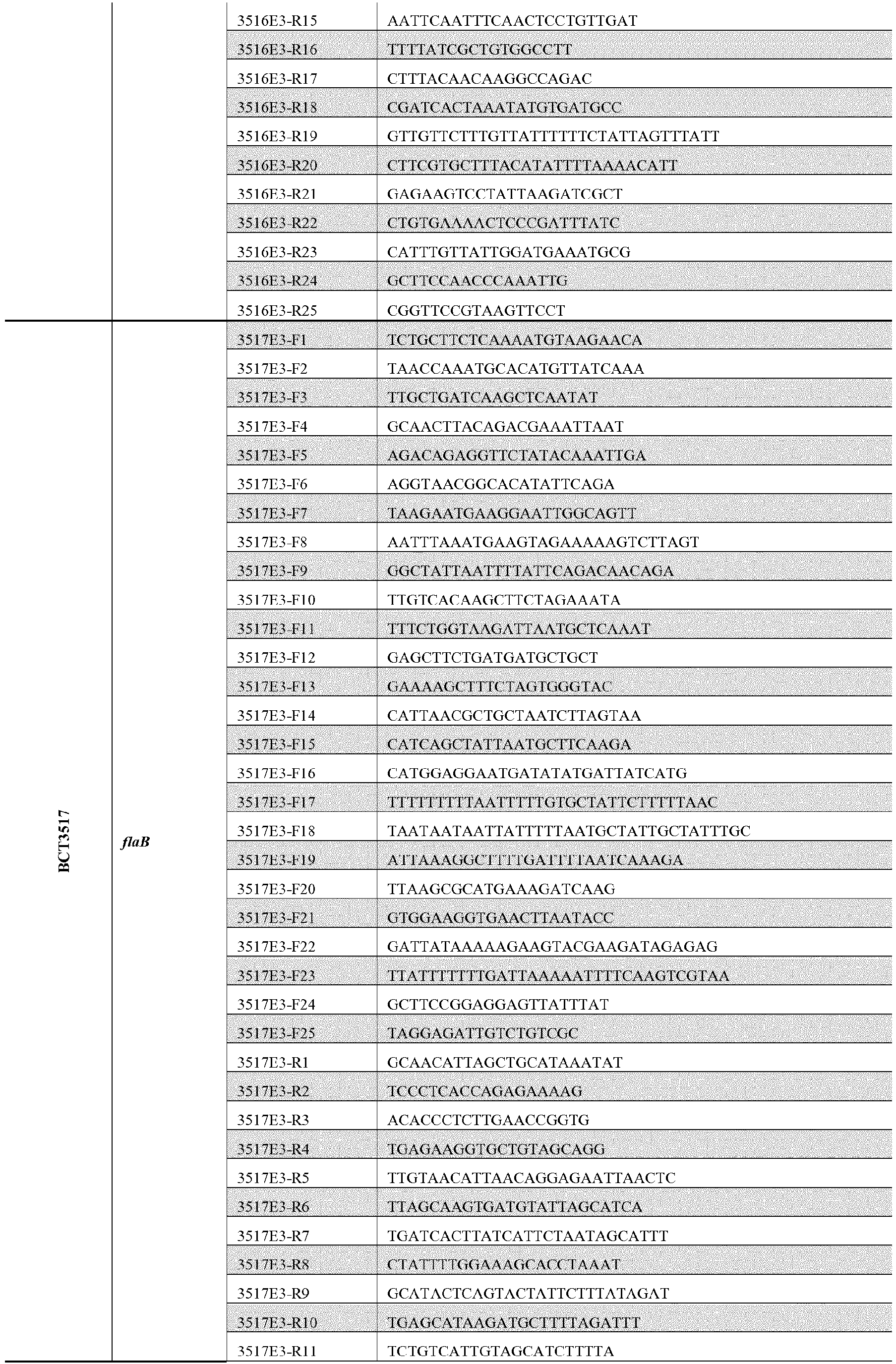

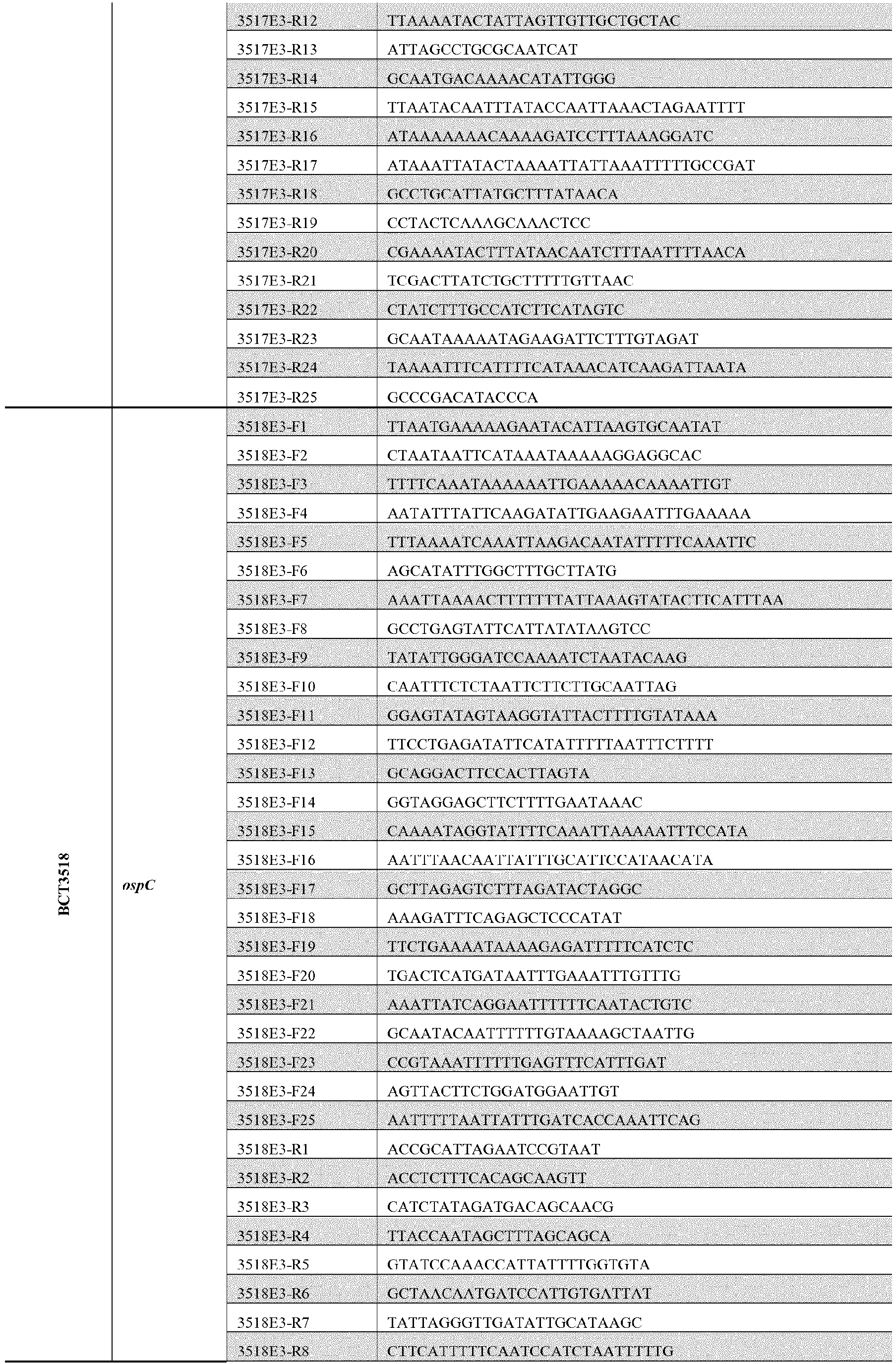

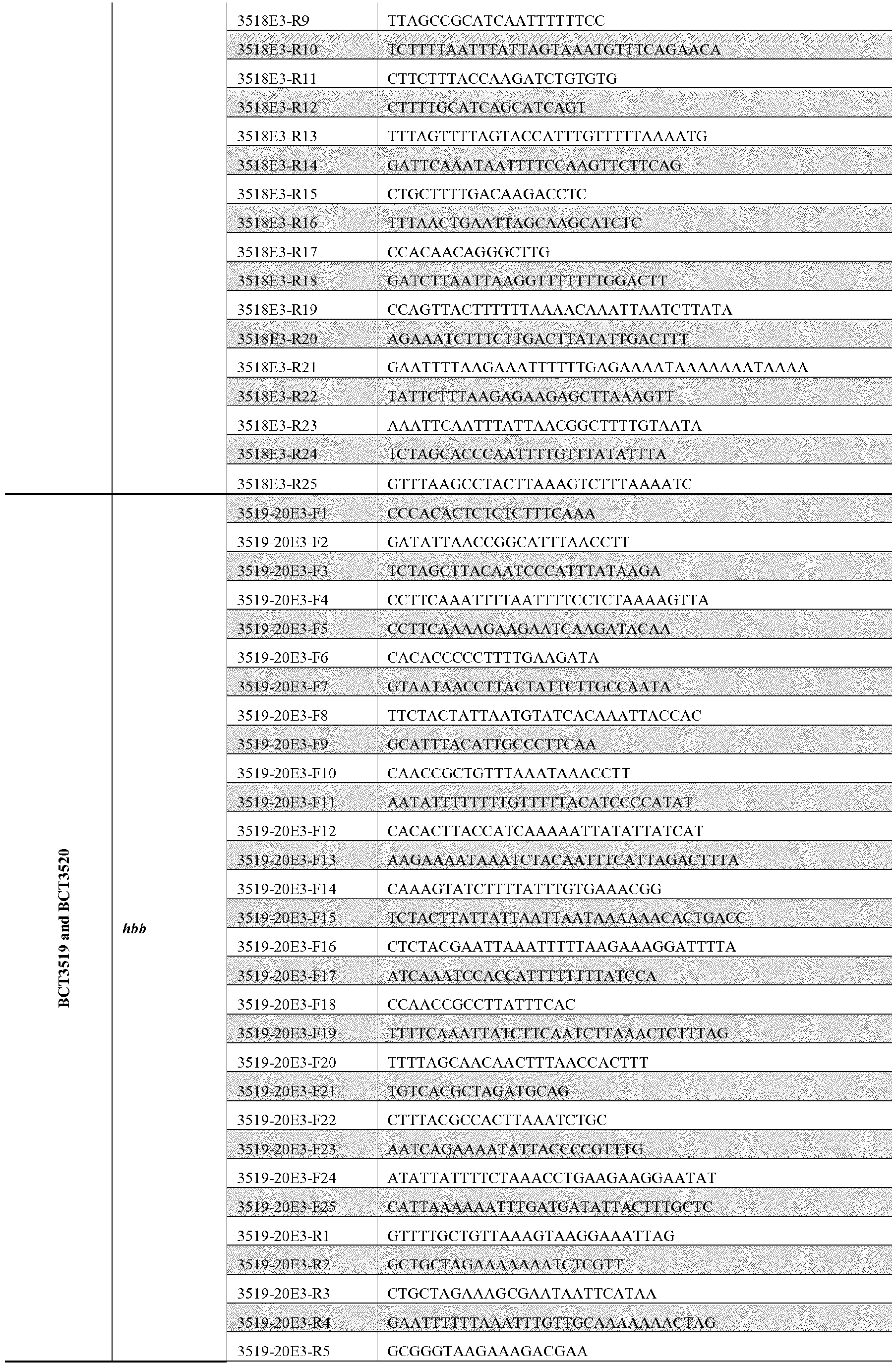

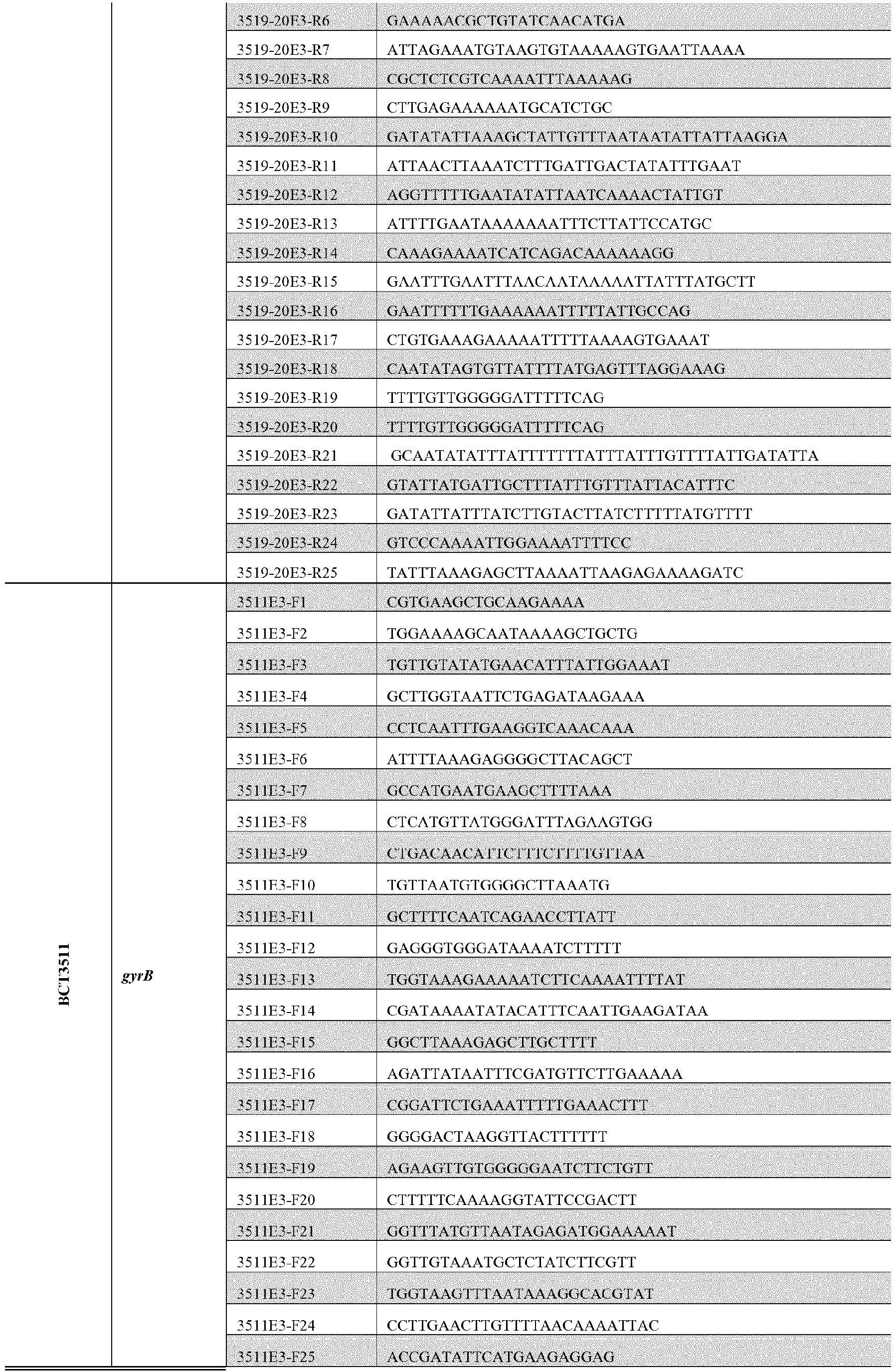

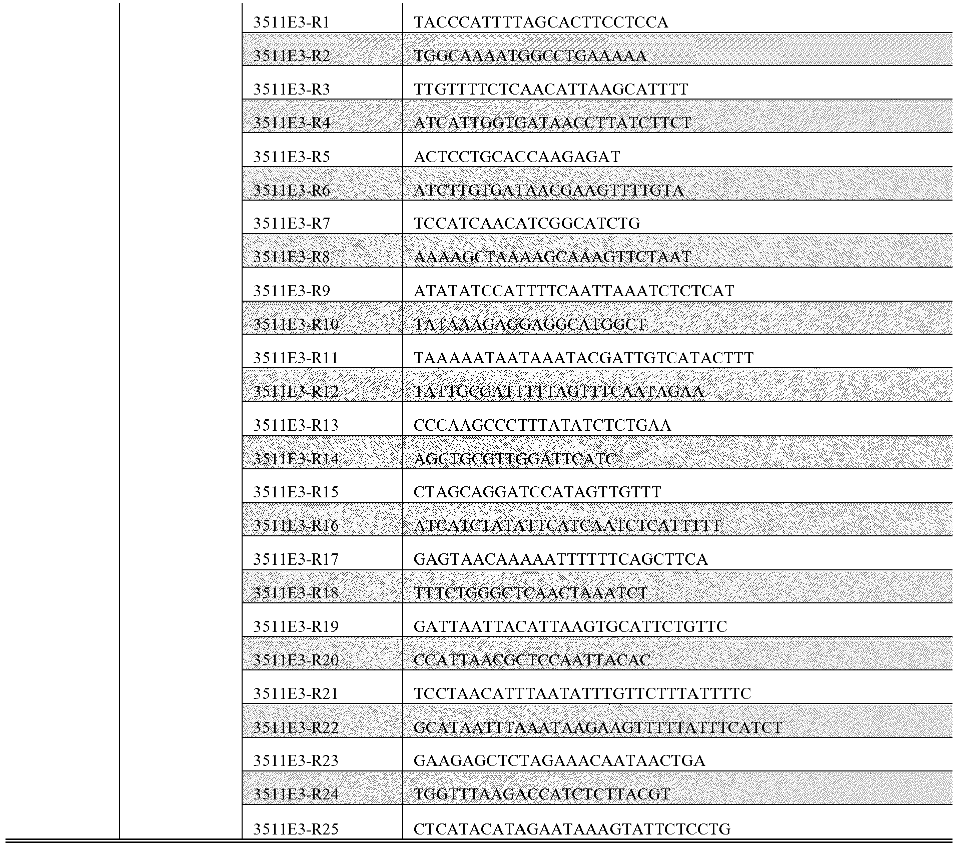

Since two of the target regions are close a single set of flanking oligos was designed to cover both targets. All primers were brought up to an initial concentration of 1mM in 10mM Tris pH 8.0 and 50uM EDTA (pH 8.0). The primers were mixed in equal proportions to create a 1mM oligo mix. Primers were designed using *B. burgdorferi* B31 genome sequence (gi|15594346) and have a GC content of 25 to 50%, spaced 6-10 nucleotides apart and have a target TM of 52°C. To ensure removal of any contaminating salts the pooled primers were dialyzed twice for 4 hours at 4°C against a 4L solution containing 10mM Tris pH 8.0 and 50uM EDTA pH 8.0 using a 5mL Float-A-Lyzer G2 (Spectrum Laboratories, Rancho Dominguez, CA) with a molecular weight cutoff of 0.5-1kDa.

The nested isothermal amplification was performed in a 225ul reaction in a 0.6mL PCR tube (Axygen Inc., Union City, California) containing 200uL of nucleic acid extract, 22.5ul Ibis 10X PCR Buffer II ²¹ (Ibis Biosciences, Carlsbad, CA), 0.2uM dNTPs (Bioline, Tauton, MA), and 10uM oligo mix. The reactions were incubated at 95°C for 10min followed by a cooling to 56°C in a MJ Thermocycler (Bio-Rad Laboratories, Hercules, CA.). The reactions were then removed to a heat block at 56°C and 11.25U of BstE DNA polymerase (Lucigen, Middleton, WI) enzyme added and the reactions incubated at 56°C for 2 hours followed by an 80°C heat inactivation for 20min. The resulting reaction was used directly in the subsequent PCR without purification.

### Detection of B. burgdorferi from whole blood from patients with clinically diagnosed early Lyme disease.

Borrelia was detected by processing 2ul of *Borrelia* enriched nucleic acid extracts per PCR reaction on a previously described broad-range PCR/ESI-MS assay designed to detect and characterize *Borrelia burgdorferi* as previously described. ²¹. Electrospray ionization mass spectrometry was performed on the PLEX-ID biosensor (Abbott Molecular). Briefly, after PCR amplification, 30 µL aliquots of each PCR reaction were desalted and analyzed by mass spectrometry as previously described ²¹⁻²³. Analysis of amplicons from any one of the eight primer pairs in the assay can be used to positively identify *Borrelia* DNA in a specimen.

### RESULTS

The data are supportive that this new strategy can positively impact current clinical and laboratory impediments to early diagnosis of Lyme disease. A serendipitous finding of clinical importance was that the majority (∼60%), of rashes were not the classic EM in PCR positive cases.

### Detection of Lyme disease in 29 patients with classic and non-classic EM

Examination of nucleic acid extract from 1.25 ml of whole blood from the independent set of 29 endemic patients with clinically diagnosed Lyme disease with classic or non-classic EM and 44 healthy controls showed that 14 of the 29 Lyme patients, and 0 of 45 controls, (48%, p=0.0001) had detectable *B. burgdorferi.* The pre-PCR enrichment step enabled increased detection of *Borrelia* from 2 to 14 cases (7X) when assessed by the PCR/ESI-MS assay. Two-tiered serology was positive in 14 of the 29 specimens, not all of which overlapped with the 14 specimens positive by NIA/PCR/ESI-MS from Table 2. Nine of the clinically defined early Lyme disease specimens tested negative by both PCR and serology assays. However, we did not have skin isolates to determine if the *Borrelia* was actually present as a skin-restricted strain which did not disseminate to blood.^{23,24} Table 2 is presented to show PCR detection is possible even when serology is negative or not yet positive. The data support the concept that unambiguous diagnosis can be made early, before seroconversion.

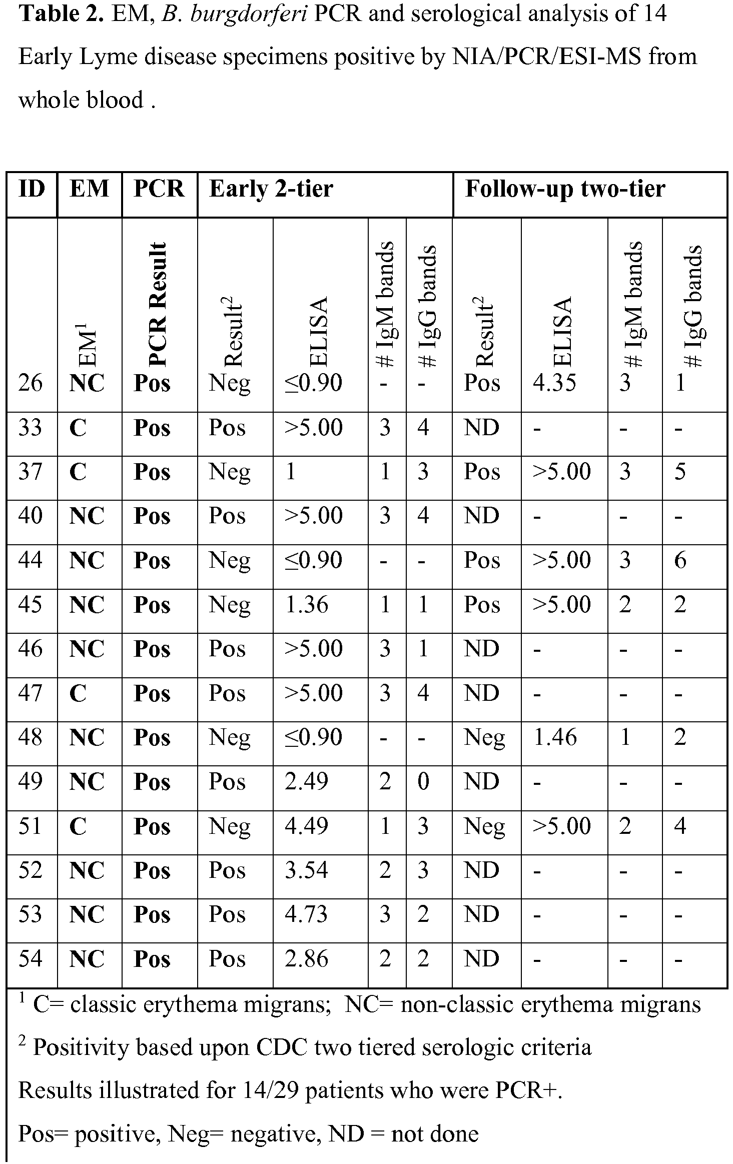

Of the 14 early Lyme specimens that tested positive by the NIA/PCR/ESI-MS assay, 6 were negative by the 2-tiered test at the time of presentation before the initiation of antibiotics (Table 2). Of these 6 specimens, 4 seroconverted by the follow-up serological testing 3 weeks later indicating that these were likely recent infections. Two of the NIA/PCR/ESI-MS positive specimens (#48 and #51) did not seroconvert sufficiently to meet the CDC surveillance criteria²⁵ but both specimens showed an increase in ELISA titers and increased IgG/IgM blot reactivity, indicating that these patients were most likely represent weak or slow responders possibly due to antibiotic therapy²⁶ or for unknown reasons. Of the 44 control patient sera, a single sample was seropositive by ELISA and IgG western blot, but was PCR negative. This low rate in our sample set from the eastern United States is consistent with the known background seroreactivity from remote exposure in an endemic region.

These results demonstrate that *B. burgdorferi* infection can be diagnosed early, and even prior to seroconversion, from whole blood in a high percentage of patients with suspected Lyme using a pre-PCR Borrelia-DNA enhancing method coupled with PCR/ESI-MS assay. Other isothermal amplification methods to generate genetic material has been described but not for Borrelia.^{7,8} In this case, a novel nested isothermal amplification (NIA) was coupled with a PCR/ESI-MS assay and results were contrasted with the two-tier serology. However, in a clinical situation, finding *Borrelia* DNA circulating in blood in an ill person with endemic area exposure will have such a strong possibility of a current infection that it would support a clinical decision to treat regardless of whether there is a classical EM, an atypical skin lesion, or extracutaneous systemic symptoms and signs suggestive of Lyme disease. The could allow for early diagnosis of the 20-30% of patients with early Lyme disease that present with "viral-like" symptoms indistinguishable for other community acquired infectious diseases. In contrast, a single serologic test, even if positive, may be difficult to interpret in a person living in an endemic area because this *single* result could represent past exposure. The present strategy has the potential to remove this ambiguity.

Work conducted during development of embodiments of the present invention led to the conclusion that whole blood may be preferable to serum or plasma for detection of *Borrelia.* While not limited to any mechanism, this may be due to the pathogen adhering to particular blood cells. In contrast to many diagnostics based on analysis of small (ul) of samples, the present disclosure is configured to handle larger volumes (mls), but within clinical practice volumes, to increase the detection of pathogens that may be present only in low copy numbers. A recent paper²⁷ showed that *B. burgdorferi* may be present in a high percentage of patients with Lyme disease but may require "teasing" it out with combinations of cultures and PCR. Although this method is not as easily performed within a clinically useful time period, as that which is described herein, it does suggest that there is an attainable target should it be present.

A serendipitous observation in this Example and an important clinical finding was the high percentage (57%) of PCR positive patients whose skin rash was not the classical EM but an atypical lesion (see Figure 1) that could be easily misdiagnosed. Photographs of lesions (all greater than 5 cm) were presented to four physicians experienced with Lyme disease, including two dermatologists. Without knowledge of the laboratory data they were asked to categorize the lesions into 1) those that they would expect any reasonably trained physician to recognize as EM and 2) into those that they would not fault such a physician from referring the patient or desiring supportive laboratory tests or more time for observation.^{28,29} Based on previous reports, including a vaccine trial, of atypical rashes in 25-30% of microbiologically confirmed *B. burgdorferi* infected subjects^{4,5} it was expected a similar percentage, but were surprised by almost 60%. This high percentage serves as an alert for physicians to consider Lyme disease in the differential diagnosis of patients with endemic area exposure and a rash that is not the classic EM. These atypical lesions serve as a reminder of the need for diagnostics for the endemic area patient who only has a flu-like illness during the high season for Lyme disease. Such a patient may benefit from the testing strategy described in this Example or, at the very least, closer monitoring.

It is believed that these results demonstrate a useful strategy to the diagnosis of infections when there is a paucity of pathogens or the immune response cannot provide an unambiguous result. It is likely that many of these difficult to diagnose infections will require a battery of diagnostic platforms each with advantages depending on when the patient presents.

### REFERENCES

1 Benach JL, Bosler EM, Hanrahan JP et al. Spirochetes isolated from the blood of two patients with Lyme disease. N Engl J Med 1983;308:740-2.
2 Steere AC. Lyme disease. N Engl J Med 1989;321:586-96.
3 Steere AC. Lyme disease. N Engl J Med 2001;345:115-25.
4 Steere AC, Sikand VK, Meurice F et al. Vaccination against Lyme disease with recombinant Borrelia burgdorferi outer-surface lipoprotein A with adjuvant. Lyme Disease Vaccine Study Group. N Engl J Med 1998;339:209-15.
5 Berger BW, Johnson RC, Kodner C, Coleman L. Cultivation of Borrelia burgdorferi from erythema migrans lesions and perilesional skin. J Clin Microbiol 1992;30:359-61.
6 Crowder CD, Rounds MA, Phillipson CA et al. Extraction of total nucleic acids from ticks for the detection of bacterial and viral pathogens. J Med Entomol 2010;47:89-94.
7 Mori Y, Notomi T. Loop-mediated isothermal amplification (LAMP): a rapid, accurate, and cost-effective diagnostic method for infectious diseases. J Infect Chemother 2009;15:62-9.
8 Notomi T, Okayama H, Masubuchi H et al. Loop-mediated isothermal amplification of DNA. Nucleic Acids Res 2000;28:E63.
9 Crowder CD, Matthews HE, Schutzer S et al. Genotypic variation and mixtures of Lyme Borrelia in Ixodes ticks from North America and Europe. PLoS ONE 2010;5:e10650.
10 Cox-Foster DL, Conlan S, Holmes EC et al. A metagenomic survey of microbes in honey bee colony collapse disorder. Science 2007;318:283-7.
11 Briese T, Palacios G, Kokoris M et al. Diagnostic system for rapid and sensitive differential detection of pathogens. Emerg Infect Dis 2005;11:310-3.
12 Eckburg PB, Bik EM, Bernstein CN et al. Diversity of the human intestinal microbial flora. Science 2005;308:1635-8.
13 Ecker DJ, Sampath R, Blyn LB et al. Rapid identification and strain-typing of respiratory pathogens for epidemic surveillance. Proc Natl Acad Sci U S A 2005;102:8012-7.
14 Hofstadler SA, Sampath R, Blyn LB et al. TIGER: the universal biosensor. Intl J Mass Spectrometry 2005;242:23-41.
15 Jacobs MR, Bajaksouzian S, Bonomo RA et al. Occurrence, distribution and origins of serotype 6C Streptococcus pneumoniae, a recently recognized serotype. J Clin Microbiol 2008.
16 Ecker DJ, Sampath R, Massire C et al. Ibis T5000: a universal biosensor approach for microbiology. Nat Rev Microbiol 2008;6:553-8.
17 Hannis JC, Manalili SM, Hall TA et al. High-resolution genotyping of Campylobacter species by use of PCR and high-throughput mass spectrometry. J Clin Microbiol 2008;46:1220-5.
18 Blyn LB, Hall TA, Libby B et al. Rapid detection and molecular serotyping of adenovirus by use of PCR followed by electrospray ionization mass spectrometry. J Clin Microbiol 2008;46:644-51.
19 Eshoo MW, Whitehouse CA, Zoll ST et al. Direct broad-range detection of alphaviruses in mosquito extracts. Virology 2007;368:286-95.
20 Sampath R, Russell KL, Massire C et al. Global surveillance of emerging Influenza virus genotypes by mass spectrometry. PLoS ONE 2007;2:e489.
21 Sampath R, Hall TA, Massire C et al. Rapid identification of emerging infectious agents using PCR and electrospray ionization mass spectrometry. Ann N Y Acad Sci 2007;1102:109-20.
22 Sampath R, Hofstadler SA, Blyn LB et al. Rapid identification of emerging pathogens: coronavirus. Emerg Infect Dis 2005;11:373-9.
23 Seinost G, Dykhuizen DE, Dattwyler RJ et al. Four clones of Borrelia burgdorferi sensu stricto cause invasive infection in humans. Infect Immun 1999;67:3518-24.
24 Wormser GP, Liveris D, Nowakowski J et al. Association of specific subtypes of Borrelia burgdorferi with hematogenous dissemination in early Lyme disease. J Infect Dis 1999;180:720-5.
25 CDC. Recommendations for test performance and interpretation from the Second National Conference on Serologic Diagnosis of Lyme Disease. MMWR Morb Mortal Wkly Rep 1995;44:590-1.
26 Dattwyler RJ, Volkman DJ, Luft BJ, Halperin JJ, Thomas J, Golightly MG. Seronegative Lyme disease. Dissociation of specific T- and B- lymphocyte responses to Borrelia burgdorferi. N Engl J Med 1988;319:1441-6.
27 Liveris D, Schwartz I, Bittker S et al. Improving the Yield of Blood Cultures in Early Lyme Disease. Journal of Clinical Microbiology 2011;JCM.
28 Berger BW. Erythema migrans. Clin Dermatol 1993;11:359-62.
29 Berger BW. Dermatologic manifestations of Lyme disease. Rev Infect Dis 1989;11 Suppl 6:S1475-S1481.

Various modification and variation of the described methods of the invention will be apparent to those skilled in the art. Although the invention has been described in connection with specific preferred embodiments, it should be understood that the invention as claimed should not be unduly limited to such specific embodiments. Indeed, various modifications of the described modes for carrying out the invention that are obvious to those skilled in the relevant fields are intended to be within the scope of the following claims.

## Claims

1. A method of detecting Lyme disease, comprising:
(1) a method of nucleic acid extraction comprising:
a) contacting a sample of whole blood with beads, a proteinase, and an anionic surfactant to generate a treated sample;
b) homogenizing said treated sample to generate a cell lysate;
c) centrifuging said cell lysate comprising a supernatant;
d) separating said supernatant from said cell lysate;
e) adding magnetic particles and lysis buffer to said supernatant to generate a magnetic-particle sample, wherein said magnetic particles are configured to bind nucleic acid molecules;
f) washing said magnetic-particle sample with a wash buffer;
g) treating said magnetic-particle sample in order to generate a dried magnetic bead sample; and
h) treating said dried magnetic bead sample with an elution buffer such that a purified nucleic acid sample is generated that comprises purified nucleic acid; and
(2) a nested isothermal amplification method comprising:
a) contacting the purified nucleic acid sample with a buffer, dNTPs, and a plurality of nested PCR primer pairs configured to amplify at least part of at least one nucleic acid sequence from *Borrelia burgdorferi*;
b) incubating said purified nucleic acid sample with a DNA polymerase under isothermal conditions such that amplified nucleic acid is generated;
c) inactivating said DNA polymerase; and
d) subjecting said amplified nucleic acid to mass spectrometry to identify *Borrelia burgdorferi* in the sample.

2. The method of Claim 1, wherein said mass spectrometry bioagent analysis comprises electrospray ionization mass spectrometry and base composition analysis.

3. The method of Claim 1, wherein said DNA polymerase comprises BstE DNA polymerase.

4. The method of Claim 1, wherein said incubating is conducted at about 56 degrees Celsius.

5. The method of Claim 1, wherein said inactivating said DNA polymerase comprises heating said sample to at least about 80 degrees Celsius.

6. The method of Claim 1, wherein said plurality of nested PCR primer pairs comprises at least 10 primer pairs.

7. The method of Claim 1, wherein said plurality of nested PCR primer pairs comprises at least 20 primer pairs.

8. The method of Claim 1, wherein said at least one bioagent target sequence comprises at least five bioagent target sequences.

9. The method of Claim 1, further comprising a step after c) but before d) of further amplifying said amplified nucleic acid without any purification of said amplified nucleic acid.

10. The method of Claim 1, wherein the sample of whole blood is from a patient with skin lesions and early Lyme disease.

11. The method of Claim 1, wherein the sample of whole blood is from a patient without classic erythema migrans (EM) skin lesions.

12. The method of any preceding claim, wherein the sample of whole blood is 1.25mL of whole blood.

## Patentansprüche

1. Verfahren zum Nachweis von Lyme-Krankheit, das umfasst:
(1) ein Verfahren der Nukleinsäureextraktion, das umfasst:
a) in Kontakt bringen einer Vollblutprobe mit Perlen, einer Proteinase und einem anionischen oberflächenaktiven Mittel, um eine behandelte Probe zu erzeugen;
b) Homogenisieren der behandelten Probe, um ein Zelllysat zu erzeugen;
c) Zentrifugieren des Zelllysats, das einen Überstand umfasst;
d) Trennen des Überstands von dem Zelllysat;
e) Zugeben von Magnetpartikeln und Lysepuffer zu dem Überstand, um eine Magnetpartikelprobe zu erzeugen, wobei die Magnetpartikel so gestaltet sind, dass sie Nukleinsäuremoleküle binden;
f) Waschen der Magnetpartikelprobe mit einem Waschpuffer;
g) Behandeln der Magnetpartikelprobe, um eine getrocknete Magnetpartikelprobe zu erzeugen; und
h) Behandeln der getrockneten Magnetpartikelprobe mit einem Elutionspuffer, sodass eine gereinigte Nukleinsäureprobe erzeugt wird, die gereinigte Nukleinsäure umfasst; und
(2) ein ineinandergeschachteltes isothermes Amplifikationsverfahren, das umfasst:
a) in Kontakt bringen der gereinigten Nukleinsäureprobe mit einem Puffer, dNTPs und einer Vielzahl von ineinandergeschachtelten PCR-Primer-Paaren, die so gestaltet sind, dass sie mindestens einen Teil von mindestens einer Nukleinsäuresequenz von *Borrelia burgdorferi* amplifizieren;
b) Inkubieren der gereinigten Nukleinsäureprobe mit einer DNA-Polymerase unter isothermen Bedingungen, sodass amplifizierte Nukleinsäure erzeugt wird;
c) Inaktivieren der DNA-Polymerase; und
d) Durchführen einer Massenspektrometrie mit der amplifizierten Nukleinsäure, um *Borrelia burgdorferi* in der Probe zu identifizieren.

2. Verfahren nach Anspruch 1, wobei die Massenspektrometrie-Analyse des biologischen Agens Elektrospray-Ionisation-Massenspektrometrie und Grundzusammensetzungsanalyse umfasst.

3. Verfahren nach Anspruch 1, wobei die DNA-Polymerase BstE-DNA-Polymerase umfasst.

4. Verfahren nach Anspruch 1, wobei das Inkubieren bei ca. 56 °C durchgeführt wird.

5. Verfahren nach Anspruch 1, wobei das Inaktivieren der DNA-Polymerase das Erhitzen der Probe auf mindestens ca. 80 °C umfasst.

6. Verfahren nach Anspruch 1, wobei die Vielzahl der ineinandergeschachtelten PCR-Primer-Paare mindestens 10 Primer-Paare umfasst.

7. Verfahren nach Anspruch 1, wobei die Vielzahl der ineinandergeschachtelten PCR-Primer-Paare mindestens 20 Primer-Paare umfasst.

8. Verfahren nach Anspruch 1, wobei die mindestens eine Zielsequenz des biologischen Agens mindestens fünf Zielsequenzen des biologischen Agens umfasst.

9. Verfahren nach Anspruch 1, das weiter einen Schritt nach c) aber vor d) des weiteren Amplifizierens der amplifizierten Nukleinsäure umfasst, ohne jegliche Reinigung der amplifizierten Nukleinsäure.

10. Verfahren nach Anspruch 1, wobei die Vollblutprobe von einem Patienten mit Hautläsionen und früher Lyme-Krankheit stammt.

11. Verfahren nach Anspruch 1, wobei die Vollblutprobe von einem Patienten ohne klassische Erythema-migrans (EM)-Hautläsionen stammt.

12. Verfahren nach einem der vorhergehenden Ansprüche, wobei die Vollblutprobe 1,25 ml Vollblut ist.

## Revendications

1. Procédé de détection de la maladie de Lyme, comprenant :
(1) un procédé d'extraction d'acide nucléique comprenant les étapes consistant à :
a) mettre en contact un échantillon de sang entier avec des billes, une protéinase et un surfactant anionique pour générer un échantillon traité ;
b) homogénéiser ledit échantillon traité pour générer un lysat de cellules ;
c) centrifuger ledit lysat de cellules comprenant un surnageant ;
d) séparer ledit surnageant à partir dudit lysat de cellules ;
e) ajouter des particules magnétiques et un tampon pour lyse au dit surnageant afin de générer un échantillon à particules magnétiques, dans lequel lesdites particules magnétiques sont configurées pour se lier à des molécules d'acide nucléique ;
f) laver ledit échantillon à particules magnétiques avec un tampon de lavage ;
g) traiter ledit échantillon à particules magnétiques dans le but de générer un échantillon séché à billes magnétiques ; et
h) traiter ledit échantillon séché à billes magnétiques avec un tampon d'élution, de sorte qu'un échantillon d'acide nucléique purifié soit généré, lequel comprend un acide nucléique purifié ; et
(2) un procédé d'amplification nichée isotherme comprenant les étapes consistant à :
a) mettre en contact l'échantillon d'acide nucléique purifié avec un tampon, des dNTP et une pluralité de paires d'amorces d'ACP nichée, configurées pour amplifier au moins une partie d'au moins une séquence d'acide nucléique provenant de *Borrelia burgdorferi* ;
b) incuber ledit échantillon d'acide nucléique purifié avec une ADN polymérase dans des conditions isothermes, de sorte qu'un acide nucléique amplifié soit généré ;
c) inactiver ladite ADN polymérase ; et
d) soumettre ledit acide nucléique amplifié à une spectrométrie de masse pour identifier *Borrelia burgdorferi* dans l'échantillon.

2. Procédé selon la revendication 1, dans laquelle ladite analyse de bio-agent par spectrométrie de masse comprend une spectrométrie de masse à ionisation par électro-pulvérisation et une analyse de la composition de base.

3. Procédé selon la revendication 1, dans laquelle ladite ADN polymérase comprend une ADN polymérase BstE.

4. Procédé selon la revendication 1, dans laquelle ladite incubation est conduite à environ 56 degrés Celsius.

5. Procédé selon la revendication 1, dans laquelle ladite inactivation de ladite ADN polymérase comprend un chauffage dudit échantillon jusqu'à au moins 80 degrés Celsius environ.

6. Procédé selon la revendication 1, dans laquelle ladite pluralité de paires d'amorces d'ACP nichée comprend au moins 10 paires d'amorces.

7. Procédé selon la revendication 1, dans laquelle ladite pluralité de paires d'amorces d'ACP nichée comprend au moins 20 paires d'amorces.

8. Procédé selon la revendication 1, dans laquelle ladite au moins une séquence cible de bio-agent comprend au moins cinq séquences cibles de bio-agent.

9. Procédé, selon la revendication 1, comprenant en outre une étape, après le paragraphe c) mais avant le d), d'amplification en outre dudit acide nucléique amplifié sans purification quelconque dudit acide nucléique amplifié.

10. Procédé selon la revendication 1, dans laquelle l'échantillon de sang entier provient d'un patient ayant des lésions cutanées et une maladie de Lyme précoce.

11. Procédé selon la revendication 1, dans laquelle l'échantillon de sang entier provient d'un patient dépourvu de lésions cutanées d'un érythème migrant (EM) classique.

12. Procédé selon l'une quelconque des revendications précédentes, dans laquelle l'échantillon de sang entier est de 1,25 mL de sang entier.


### SEQUENCE LISTING

<110> IBIS BIOSCIENCES, INC.
<120> SAMPLE PREPARATION METHODS
<130> 11211WOO1
<140> PCT/US2012/053909
   <141> 2012-09-06
<150> 61/531,471
   <151> 2011-09-06
<160> 350
<170> PatentIn version 3.5
<210> 1
   <211> 19
   <212> DNA
   <213> Artificial Sequence
<220>
   <223> Description of Artificial Sequence: Synthetic primer
<400> 1
   ccgaaaaaga tgggctttt 19
<210> 2
   <211> 23
   <212> DNA
   <213> Artificial Sequence
<220>
   <223> Description of Artificial Sequence: Synthetic primer
<400> 2
   aggttaaaaa gtccgaaact att 23
<210> 3
   <211> 23
   <212> DNA
   <213> Artificial Sequence
<220>
   <223> Description of Artificial Sequence: Synthetic primer
<400> 3
   tctcccgatc aaattagaaa ttg 23
<210> 4
   <211> 29
   <212> DNA
   <213> Artificial Sequence
<220>
   <223> Description of Artificial Sequence: Synthetic primer
<400> 4
   aaagagataa aagattttga aagaataaa 29
<210> 5
   <211> 22
   <212> DNA
   <213> Artificial Sequence
<220>
   <223> Description of Artificial Sequence: Synthetic primer
<400> 5
   aaagctaggt ttttggagtt tt 22
<210> 6
   <211> 26
   <212> DNA
   <213> Artificial Sequence
<220>
   <223> Description of Artificial Sequence: Synthetic primer
<400> 6
   acagaaaaag aagaagaatt gattaa 26
<210> 7
   <211> 21
   <212> DNA
   <213> Artificial Sequence
<220>
   <223> Description of Artificial Sequence: Synthetic primer
<400> 7
   atgatgctgg gaatcaggtt c 21
<210> 8
   <211> 18
   <212> DNA
   <213> Artificial Sequence
<220>
   <223> Description of Artificial Sequence: Synthetic primer
<400> 8
   gggcttggac ttgatttg 18
<210> 9
   <211> 24
   <212> DNA
   <213> Artificial Sequence
<220>
   <223> Description of Artificial Sequence: Synthetic primer
<400> 9
   gtcttttaat gtgctaatgc aaga 24
<210> 10
   <211> 23
   <212> DNA
   <213> Artificial Sequence
<220>
   <223> Description of Artificial Sequence: Synthetic primer
<400> 10
   gcgttcctac taatgtatca ggg 23
<210> 11
   <211> 28
   <212> DNA
   <213> Artificial Sequence
<220>
   <223> Description of Artificial Sequence: Synthetic primer
<400> 11
   ggcagagtta aaatatatga aaatatag 28
<210> 12
   <211> 23
   <212> DNA
   <213> Artificial Sequence
<220>
   <223> Description of Artificial Sequence: Synthetic primer
<400> 12
   cacccttcaa gaacttttaa cag 23
<210> 13
   <211> 18
   <212> DNA
   <213> Artificial Sequence
<220>
   <223> Description of Artificial Sequence: Synthetic primer
<400> 13
   ggctcttgaa gcttatgg 18
<210> 14
   <211> 19
   <212> DNA
   <213> Artificial Sequence
<220>
   <223> Description of Artificial Sequence: Synthetic primer
<400> 14
   agacttggag aaatggagg 19
<210> 15
   <211> 19
   <212> DNA
   <213> Artificial Sequence
<220>
   <223> Description of Artificial Sequence: Synthetic primer
<400> 15
   gaggaaaggc tcaatttgg 19
<210> 16
   <211> 19
   <212> DNA
   <213> Artificial Sequence
<220>
   <223> Description of Artificial Sequence: Synthetic primer
<400> 16
   tcttgtttct cagcaacct 19
<210> 17
   <211> 16
   <212> DNA
   <213> Artificial Sequence
<220>
   <223> Description of Artificial Sequence: Synthetic primer
<400> 17
   cgcaagatca acaggc 16
<210> 18
   <211> 23
   <212> DNA
   <213> Artificial Sequence
<220>
   <223> Description of Artificial Sequence: Synthetic primer
<400> 18
   actacaccat cttgttgatg ata 23
<210> 19
   <211> 21
   <212> DNA
   <213> Artificial Sequence
<220>
   <223> Description of Artificial Sequence: Synthetic primer
<400> 19
   gtaatggttg gggtgattta c 21
<210> 20
   <211> 16
   <212> DNA
   <213> Artificial Sequence
<220>
   <223> Description of Artificial Sequence: Synthetic primer
<400> 20
   ggagagccgt tcgaaa 16
<210> 21
   <211> 29
   <212> DNA
   <213> Artificial Sequence
<220>
   <223> Description of Artificial Sequence: Synthetic primer
<400> 21
   ccaacttcta aagaaatttt atatgatgg 29
<210> 22
   <211> 23
   <212> DNA
   <213> Artificial Sequence
<220>
   <223> Description of Artificial Sequence: Synthetic primer
<400> 22
   aggaaaaatt aaaaactgct gga 23
<210> 23
   <211> 24
   <212> DNA
   <213> Artificial Sequence
<220>
   <223> Description of Artificial Sequence: Synthetic primer
<400> 23
   tgtttttgaa tctgctacaa atga 24
<210> 24
   <211> 28
   <212> DNA
   <213> Artificial Sequence
<220>
   <223> Description of Artificial Sequence: Synthetic primer
<400> 24
   ctggtaaata tcttggtgaa tcttataa 28
<210> 25
   <211> 22
   <212> DNA
   <213> Artificial Sequence
<220>
   <223> Description of Artificial Sequence: Synthetic primer
<400> 25
   ggacagttaa tggaatctca at 22
<210> 26
   <211> 24
   <212> DNA
   <213> Artificial Sequence
<220>
   <223> Description of Artificial Sequence: Synthetic primer
<400> 26
   ataccaaata tgagcaactg gggc 24
<210> 27
   <211> 20
   <212> DNA
   <213> Artificial Sequence
<220>
   <223> Description of Artificial Sequence: Synthetic primer
<400> 27
   aagcccaatc ctagagggta 20
<210> 28
   <211> 25
   <212> DNA
   <213> Artificial Sequence
<220>
   <223> Description of Artificial Sequence: Synthetic primer
<400> 28
   tagaattcaa actagatgct gtaat 25
<210> 29
   <211> 27
   <212> DNA
   <213> Artificial Sequence
<220>
   <223> Description of Artificial Sequence: Synthetic primer
<400> 29
   cggttcaatt actacatatt tttcata 27
<210> 30
   <211> 19
   <212> DNA
   <213> Artificial Sequence
<220>
   <223> Description of Artificial Sequence: Synthetic primer
<400> 30
   gcccggttca attactaca 19
<210> 31
   <211> 24
   <212> DNA
   <213> Artificial Sequence
<220>
   <223> Description of Artificial Sequence: Synthetic primer
<400> 31
   tcttcattta aaagctgcat tttt 24
<210> 32
   <211> 23
   <212> DNA
   <213> Artificial Sequence
<220>
   <223> Description of Artificial Sequence: Synthetic primer
<400> 32
   gctctctagc ttctatgtac tca 23
<210> 33
   <211> 26
   <212> DNA
   <213> Artificial Sequence
<220>
   <223> Description of Artificial Sequence: Synthetic primer
<400> 33
   aagcattaaa agacatacca tatcgc 26
<210> 34
   <211> 24
   <212> DNA
   <213> Artificial Sequence
<220>
   <223> Description of Artificial Sequence: Synthetic primer
<400> 34
   gaagagtttt aatagcctca gccc 24
<210> 35
   <211> 21
   <212> DNA
   <213> Artificial Sequence
<220>
   <223> Description of Artificial Sequence: Synthetic primer
<400> 35
   gacgaaagct catcaagatc a 21
<210> 36
   <211> 30
   <212> DNA
   <213> Artificial Sequence
<220>
   <223> Description of Artificial Sequence: Synthetic primer
<400> 36
   cagttttatc atctttatct atcatttgaa 30
<210> 37
   <211> 27
   <212> DNA
   <213> Artificial Sequence
<220>
   <223> Description of Artificial Sequence: Synthetic primer
<400> 37
   aaattctcaa taatttcaag acgtctt 27
<210> 38
   <211> 21
   <212> DNA
   <213> Artificial Sequence
<220>
   <223> Description of Artificial Sequence: Synthetic primer
<400> 38
   atccactctg gcttattgcc a 21
<210> 39
   <211> 19
   <212> DNA
   <213> Artificial Sequence
<220>
   <223> Description of Artificial Sequence: Synthetic primer
<400> 39
   ggggaataac aggaagaac 19
<210> 40
   <211> 15
   <212> DNA
   <213> Artificial Sequence
<220>
   <223> Description of Artificial Sequence: Synthetic primer
<400> 40
   gctgaaccat tggcc 15
<210> 41
   <211> 15
   <212> DNA
   <213> Artificial Sequence
<220>
   <223> Description of Artificial Sequence: Synthetic primer
<400> 41
   atgttgcaaa gcgcc 15
<210> 42
   <211> 35
   <212> DNA
   <213> Artificial Sequence
<220>
   <223> Description of Artificial Sequence: Synthetic primer
<400> 42
   cgattatttc tatttatgac tcttctataa agatc 35
<210> 43
   <211> 23
   <212> DNA
   <213> Artificial Sequence
<220>
   <223> Description of Artificial Sequence: Synthetic primer
<400> 43
   gcattaagaa gaagcaactt tct 23
<210> 44
   <211> 27
   <212> DNA
   <213> Artificial Sequence
<220>
   <223> Description of Artificial Sequence: Synthetic primer
<400> 44
   attctttttt cgtttctcac aataatc 27
<210> 45
   <211> 24
   <212> DNA
   <213> Artificial Sequence
<220>
   <223> Description of Artificial Sequence: Synthetic primer
<400> 45
   gtcaaaaaga gagtctactg attc 24
<210> 46
   <211> 24
   <212> DNA
   <213> Artificial Sequence
<220>
   <223> Description of Artificial Sequence: Synthetic primer
<400> 46
   gaacctttga caacctttct ttta 24
<210> 47
   <211> 15
   <212> DNA
   <213> Artificial Sequence
<220>
   <223> Description of Artificial Sequence: Synthetic primer
<400> 47
   cgacttgaga ggcct 15
<210> 48
   <211> 21
   <212> DNA
   <213> Artificial Sequence
<220>
   <223> Description of Artificial Sequence: Synthetic primer
<400> 48
   cctgcttacc ttttaatgca t 21
<210> 49
   <211> 24
   <212> DNA
   <213> Artificial Sequence
<220>
   <223> Description of Artificial Sequence: Synthetic primer
<400> 49
   ttttaccaag aagattttgc ctaa 24
<210> 50
   <211> 23
   <212> DNA
   <213> Artificial Sequence
<220>
   <223> Description of Artificial Sequence: Synthetic primer
<400> 50
   caataacaga acgaccagaa taa 23
<210> 51
   <211> 21
   <212> DNA
   <213> Artificial Sequence
<220>
   <223> Description of Artificial Sequence: Synthetic primer
<400> 51
   gtgttgctat gaatcctgtt g 21
<210> 52
   <211> 17
   <212> DNA
   <213> Artificial Sequence
<220>
   <223> Description of Artificial Sequence: Synthetic primer
<400> 52
   ggtagaagac ccaaggt 17
<210> 53
   <211> 20
   <212> DNA
   <213> Artificial Sequence
<220>
   <223> Description of Artificial Sequence: Synthetic primer
<400> 53
   ggaaagctgg taaaagtagg 20
<210> 54
   <211> 26
   <212> DNA
   <213> Artificial Sequence
<220>
   <223> Description of Artificial Sequence: Synthetic primer
<400> 54
   tggaaatgaa gattatgcca atattt 26
<210> 55
   <211> 28
   <212> DNA
   <213> Artificial Sequence
<220>
   <223> Description of Artificial Sequence: Synthetic primer
<400> 55
   tttttaaaaa atgtattgca acaattgg 28
<210> 56
   <211> 20
   <212> DNA
   <213> Artificial Sequence
<220>
   <223> Description of Artificial Sequence: Synthetic primer
<400> 56
   ttatcatctg gcgagatgag 20
<210> 57
   <211> 19
   <212> DNA
   <213> Artificial Sequence
<220>
   <223> Description of Artificial Sequence: Synthetic primer
<400> 57
   gacgggaatt atgtcactg 19
<210> 58
   <211> 22
   <212> DNA
   <213> Artificial Sequence
<220>
   <223> Description of Artificial Sequence: Synthetic primer
<400> 58
   ggtggatatg ctatgatact tg 22
<210> 59
   <211> 19
   <212> DNA
   <213> Artificial Sequence
<220>
   <223> Description of Artificial Sequence: Synthetic primer
<400> 59
   gggtggacag cttataaga 19
<210> 60
   <211> 24
   <212> DNA
   <213> Artificial Sequence
<220>
   <223> Description of Artificial Sequence: Synthetic primer
<400> 60
   cgttcacaat attgagctta atgt 24
<210> 61
   <211> 27
   <212> DNA
   <213> Artificial Sequence
<220>
   <223> Description of Artificial Sequence: Synthetic primer
<400> 61
   cttacctctt gaaaatattc ctattgg 27
<210> 62
   <211> 23
   <212> DNA
   <213> Artificial Sequence
<220>
   <223> Description of Artificial Sequence: Synthetic primer
<400> 62
   ctaatgctcc aattaaaatt ggc 23
<210> 63
   <211> 21
   <212> DNA
   <213> Artificial Sequence
<220>
   <223> Description of Artificial Sequence: Synthetic primer
<400> 63
   ggttggagat gttttggaaa g 21
<210> 64
   <211> 26
   <212> DNA
   <213> Artificial Sequence
<220>
   <223> Description of Artificial Sequence: Synthetic primer
<400> 64
   aaaggtatat tatttctcct aaaggc 26
<210> 65
   <211> 28
   <212> DNA
   <213> Artificial Sequence
<220>
   <223> Description of Artificial Sequence: Synthetic primer
<400> 65
   gctaatatag ctttgcttgt ttataaag 28
<210> 66
   <211> 26
   <212> DNA
   <213> Artificial Sequence
<220>
   <223> Description of Artificial Sequence: Synthetic primer
<400> 66
   gttgcttcta ttgaatatga tcctaa 26
<210> 67
   <211> 25
   <212> DNA
   <213> Artificial Sequence
<220>
   <223> Description of Artificial Sequence: Synthetic primer
<400> 67
   cgaagagata aatttagcat tcctg 25
<210> 68
   <211> 26
   <212> DNA
   <213> Artificial Sequence
<220>
   <223> Description of Artificial Sequence: Synthetic primer
<400> 68
   gcataagaga aagtataggt tgattg 26
<210> 69
   <211> 27
   <212> DNA
   <213> Artificial Sequence
<220>
   <223> Description of Artificial Sequence: Synthetic primer
<400> 69
   ctggtaggat tagtattaga agaagag 27
<210> 70
   <211> 25
   <212> DNA
   <213> Artificial Sequence
<220>
   <223> Description of Artificial Sequence: Synthetic primer
<400> 70
   aaaaggtaaa aaatttaaat cgggc 25
<210> 71
   <211> 24
   <212> DNA
   <213> Artificial Sequence
<220>
   <223> Description of Artificial Sequence: Synthetic primer
<400> 71
   caaaggtaat gatcctttga aatc 24
<210> 72
   <211> 26
   <212> DNA
   <213> Artificial Sequence
<220>
   <223> Description of Artificial Sequence: Synthetic primer
<400> 72
   gctataagac gactttatct tttgat 26
<210> 73
   <211> 25
   <212> DNA
   <213> Artificial Sequence
<220>
   <223> Description of Artificial Sequence: Synthetic primer
<400> 73
   agacttataa gccaaaaact tcttc 25
<210> 74
   <211> 25
   <212> DNA
   <213> Artificial Sequence
<220>
   <223> Description of Artificial Sequence: Synthetic primer
<400> 74
   ggttttggag aaaaataaat atggg 25
<210> 75
   <211> 33
   <212> DNA
   <213> Artificial Sequence
<220>
   <223> Description of Artificial Sequence: Synthetic primer
<400> 75
   caaaaaggaa gataaaatag atatttttta gtg 33
<210> 76
   <211> 22
   <212> DNA
   <213> Artificial Sequence
<220>
   <223> Description of Artificial Sequence: Synthetic primer
<400> 76
   tttcttgcga gtcttataac ct 22
<210> 77
   <211> 38
   <212> DNA
   <213> Artificial Sequence
<220>
   <223> Description of Artificial Sequence: Synthetic primer
<400> 77
   tttatttctt cttttaataa taaatttatc tgaatatc 38
<210> 78
   <211> 28
   <212> DNA
   <213> Artificial Sequence
<220>
   <223> Description of Artificial Sequence: Synthetic primer
<400> 78
   ttttttaata gatcttgcca ctatactc 28
<210> 79
   <211> 31
   <212> DNA
   <213> Artificial Sequence
<220>
   <223> Description of Artificial Sequence: Synthetic primer
<400> 79
   actttttgat aaagactctt ttctataaaa g 31
<210> 80
   <211> 20
   <212> DNA
   <213> Artificial Sequence
<220>
   <223> Description of Artificial Sequence: Synthetic primer
<400> 80
   cttctcactt ccaaaagacg 20
<210> 81
   <211> 25
   <212> DNA
   <213> Artificial Sequence
<220>
   <223> Description of Artificial Sequence: Synthetic primer
<400> 81
   aagatctgga gtaggtttta ataac 25
<210> 82
   <211> 22
   <212> DNA
   <213> Artificial Sequence
<220>
   <223> Description of Artificial Sequence: Synthetic primer
<400> 82
   ggcttaccat ttcaggaatt at 22
<210> 83
   <211> 25
   <212> DNA
   <213> Artificial Sequence
<220>
   <223> Description of Artificial Sequence: Synthetic primer
<400> 83
   aagttttgcc attgtaaaca gatat 25
<210> 84
   <211> 26
   <212> DNA
   <213> Artificial Sequence
<220>
   <223> Description of Artificial Sequence: Synthetic primer
<400> 84
   caagatcctc ggtaatataa ataggt 26
<210> 85
   <211> 17
   <212> DNA
   <213> Artificial Sequence
<220>
   <223> Description of Artificial Sequence: Synthetic primer
<400> 85
   ctcgccaagc ttatgtc 17
<210> 86
   <211> 22
   <212> DNA
   <213> Artificial Sequence
<220>
   <223> Description of Artificial Sequence: Synthetic primer
<400> 86
   cctctaaaaa tccttgtagg tg 22
<210> 87
   <211> 22
   <212> DNA
   <213> Artificial Sequence
<220>
   <223> Description of Artificial Sequence: Synthetic primer
<400> 87
   cctctaaaaa tccttgtagg tg 22
<210> 88
   <211> 23
   <212> DNA
   <213> Artificial Sequence
<220>
   <223> Description of Artificial Sequence: Synthetic primer
<400> 88
   cttccctttt tatctgactt agc 23
<210> 89
   <211> 29
   <212> DNA
   <213> Artificial Sequence
<220>
   <223> Description of Artificial Sequence: Synthetic primer
<400> 89
   atcttctatt taccaacata actacttac 29
<210> 90
   <211> 21
   <212> DNA
   <213> Artificial Sequence
<220>
   <223> Description of Artificial Sequence: Synthetic primer
<400> 90
   agggtaaatt tttgcccttt g 21
<210> 91
   <211> 22
   <212> DNA
   <213> Artificial Sequence
<220>
   <223> Description of Artificial Sequence: Synthetic primer
<400> 91
   ctattggcct aacttttttt gg 22
<210> 92
   <211> 18
   <212> DNA
   <213> Artificial Sequence
<220>
   <223> Description of Artificial Sequence: Synthetic primer
<400> 92
   agactctccc cggatatt 18
<210> 93
   <211> 17
   <212> DNA
   <213> Artificial Sequence
<220>
   <223> Description of Artificial Sequence: Synthetic primer
<400> 93
   aaagcactgc aatagcc 17
<210> 94
   <211> 24
   <212> DNA
   <213> Artificial Sequence
<220>
   <223> Description of Artificial Sequence: Synthetic primer
<400> 94
   taaaagctta gctcctttat tagg 24
<210> 95
   <211> 20
   <212> DNA
   <213> Artificial Sequence
<220>
   <223> Description of Artificial Sequence: Synthetic primer
<400> 95
   tgatgctgct gacttaacaa 20
<210> 96
   <211> 26
   <212> DNA
   <213> Artificial Sequence
<220>
   <223> Description of Artificial Sequence: Synthetic primer
<400> 96
   ctcggaaaga tttttattgt gataca 26
<210> 97
   <211> 29
   <212> DNA
   <213> Artificial Sequence
<220>
   <223> Description of Artificial Sequence: Synthetic primer
<400> 97
   catcaaccat aactgtttta acaaatatc 29
<210> 98
   <211> 21
   <212> DNA
   <213> Artificial Sequence
<220>
   <223> Description of Artificial Sequence: Synthetic primer
<400> 98
   gccaaatctt tttacgacga c 21
<210> 99
   <211> 19
   <212> DNA
   <213> Artificial Sequence
<220>
   <223> Description of Artificial Sequence: Synthetic primer
<400> 99
   tatcagctct acccctagc 19
<210> 100
   <211> 31
   <212> DNA
   <213> Artificial Sequence
<220>
   <223> Description of Artificial Sequence: Synthetic primer
<400> 100
   cttcaacaaa aatatgacaa tttctattaa c 31
<210> 101
   <211> 18
   <212> DNA
   <213> Artificial Sequence
<220>
   <223> Description of Artificial Sequence: Synthetic primer
<400> 101
   cctatccttc tgccaacg 18
<210> 102
   <211> 28
   <212> DNA
   <213> Artificial Sequence
<220>
   <223> Description of Artificial Sequence: Synthetic primer
<400> 102
   ggaaaaaaga ttgtatatac ttgacatg 28
<210> 103
   <211> 26
   <212> DNA
   <213> Artificial Sequence
<220>
   <223> Description of Artificial Sequence: Synthetic primer
<400> 103
   caaagtagaa gaagatccaa gtattc 26
<210> 104
   <211> 24
   <212> DNA
   <213> Artificial Sequence
<220>
   <223> Description of Artificial Sequence: Synthetic primer
<400> 104
   ggcaagaatt ttgggataat aaca 24
<210> 105
   <211> 30
   <212> DNA
   <213> Artificial Sequence
<220>
   <223> Description of Artificial Sequence: Synthetic primer
<400> 105
   tgtctaaata cgaattcata aaaattgaaa 30
<210> 106
   <211> 30
   <212> DNA
   <213> Artificial Sequence
<220>
   <223> Description of Artificial Sequence: Synthetic primer
<400> 106
   gaataagttg ttatgagtta attttcaaga 30
<210> 107
   <211> 34
   <212> DNA
   <213> Artificial Sequence
<220>
   <223> Description of Artificial Sequence: Synthetic primer
<400> 107
   taaataaaat taacaaaaat gcaatctaaa agaa 34
<210> 108
   <211> 21
   <212> DNA
   <213> Artificial Sequence
<220>
   <223> Description of Artificial Sequence: Synthetic primer
<400> 108
   cacagcatca aaattgttag c 21
<210> 109
   <211> 15
   <212> DNA
   <213> Artificial Sequence
<220>
   <223> Description of Artificial Sequence: Synthetic primer
<400> 109
   ccgtgctggt tcaag 15
<210> 110
   <211> 14
   <212> DNA
   <213> Artificial Sequence
<220>
   <223> Description of Artificial Sequence: Synthetic primer
<400> 110
   gaggggctag tggg 14
<210> 111
   <211> 17
   <212> DNA
   <213> Artificial Sequence
<220>
   <223> Description of Artificial Sequence: Synthetic primer
<400> 111
   ggaagtggta gacacgc 17
<210> 112
   <211> 28
   <212> DNA
   <213> Artificial Sequence
<220>
   <223> Description of Artificial Sequence: Synthetic primer
<400> 112
   aaaaaatata atggttaata gtgctgtg 28
<210> 113
   <211> 34
   <212> DNA
   <213> Artificial Sequence
<220>
   <223> Description of Artificial Sequence: Synthetic primer
<400> 113
   gtaattaaaa aaaataaaaa agttgacaaa aatt 34
<210> 114
   <211> 25
   <212> DNA
   <213> Artificial Sequence
<220>
   <223> Description of Artificial Sequence: Synthetic primer
<400> 114
   cgctgtaata gcaacaacaa taata 25
<210> 115
   <211> 38
   <212> DNA
   <213> Artificial Sequence
<220>
   <223> Description of Artificial Sequence: Synthetic primer
<400> 115
   aataatattt tcaaaaataa aaataattat atttgcaa 38
<210> 116
   <211> 22
   <212> DNA
   <213> Artificial Sequence
<220>
   <223> Description of Artificial Sequence: Synthetic primer
<400> 116
   ctctaagctt caaactaggt ca 22
<210> 117
   <211> 23
   <212> DNA
   <213> Artificial Sequence
<220>
   <223> Description of Artificial Sequence: Synthetic primer
<400> 117
   aactttgctc tcaatagttg ttt 23
<210> 118
   <211> 32
   <212> DNA
   <213> Artificial Sequence
<220>
   <223> Description of Artificial Sequence: Synthetic primer
<400> 118
   tatgaaatct aatctattta ttgtttctga ct 32
<210> 119
   <211> 22
   <212> DNA
   <213> Artificial Sequence
<220>
   <223> Description of Artificial Sequence: Synthetic primer
<400> 119
   gcaatattta tgtcagcagg aa 22
<210> 120
   <211> 21
   <212> DNA
   <213> Artificial Sequence
<220>
   <223> Description of Artificial Sequence: Synthetic primer
<400> 120
   gaaggtttat accctttgga g 21
<210> 121
   <211> 26
   <212> DNA
   <213> Artificial Sequence
<220>
   <223> Description of Artificial Sequence: Synthetic primer
<400> 121
   tggtcaatat ggggtattaa ctttat 26
<210> 122
   <211> 27
   <212> DNA
   <213> Artificial Sequence
<220>
   <223> Description of Artificial Sequence: Synthetic primer
<400> 122
   caataacctg cttgacaaaa taaatta 27
<210> 123
   <211> 33
   <212> DNA
   <213> Artificial Sequence
<220>
   <223> Description of Artificial Sequence: Synthetic primer
<400> 123
   ggaaattaat gggaaataaa ttatttaaaa aca 33
<210> 124
   <211> 28
   <212> DNA
   <213> Artificial Sequence
<220>
   <223> Description of Artificial Sequence: Synthetic primer
<400> 124
   agacataata tctttttaca ttgggaaa 28
<210> 125
   <211> 21
   <212> DNA
   <213> Artificial Sequence
<220>
   <223> Description of Artificial Sequence: Synthetic primer
<400> 125
   tttaggaatt ttttggggag c 21
<210> 126
   <211> 17
   <212> DNA
   <213> Artificial Sequence
<220>
   <223> Description of Artificial Sequence: Synthetic primer
<400> 126
   tgagggtgag ttcctgt 17
<210> 127
   <211> 35
   <212> DNA
   <213> Artificial Sequence
<220>
   <223> Description of Artificial Sequence: Synthetic primer
<400> 127
   ttgtttttta aacttattaa tattttcttc tgtac 35
<210> 128
   <211> 15
   <212> DNA
   <213> Artificial Sequence
<220>
   <223> Description of Artificial Sequence: Synthetic primer
<400> 128
   ggcaaacccc aaagc 15
<210> 129
   <211> 21
   <212> DNA
   <213> Artificial Sequence
<220>
   <223> Description of Artificial Sequence: Synthetic primer
<400> 129
   gtgttctaat ttctcgatcc c 21
<210> 130
   <211> 25
   <212> DNA
   <213> Artificial Sequence
<220>
   <223> Description of Artificial Sequence: Synthetic primer
<400> 130
   actgtgtcca tttatagtaa ttctc 25
<210> 131
   <211> 26
   <212> DNA
   <213> Artificial Sequence
<220>
   <223> Description of Artificial Sequence: Synthetic primer
<400> 131
   ctagcccttt tttatataat tgcagg 26
<210> 132
   <211> 23
   <212> DNA
   <213> Artificial Sequence
<220>
   <223> Description of Artificial Sequence: Synthetic primer
<400> 132
   gtaccataca ggcatttctt tta 23
<210> 133
   <211> 18
   <212> DNA
   <213> Artificial Sequence
<220>
   <223> Description of Artificial Sequence: Synthetic primer
<400> 133
   ctagtaccgt tccaagct 18
<210> 134
   <211> 25
   <212> DNA
   <213> Artificial Sequence
<220>
   <223> Description of Artificial Sequence: Synthetic primer
<400> 134
   gtccatctga agtttgaata atctc 25
<210> 135
   <211> 18
   <212> DNA
   <213> Artificial Sequence
<220>
   <223> Description of Artificial Sequence: Synthetic primer
<400> 135
   acgctgtaag atcctctc 18
<210> 136
   <211> 29
   <212> DNA
   <213> Artificial Sequence
<220>
   <223> Description of Artificial Sequence: Synthetic primer
<400> 136
   gtaattttta aaacccactg tcttaaata 29
<210> 137
   <211> 19
   <212> DNA
   <213> Artificial Sequence
<220>
   <223> Description of Artificial Sequence: Synthetic primer
<400> 137
   cgtctagcaa tctttcagc 19
<210> 138
   <211> 22
   <212> DNA
   <213> Artificial Sequence
<220>
   <223> Description of Artificial Sequence: Synthetic primer
<400> 138
   agattcaggc cattctaatt ct 22
<210> 139
   <211> 19
   <212> DNA
   <213> Artificial Sequence
<220>
   <223> Description of Artificial Sequence: Synthetic primer
<400> 139
   tccaatttcg ctgcatttc 19
<210> 140
   <211> 24
   <212> DNA
   <213> Artificial Sequence
<220>
   <223> Description of Artificial Sequence: Synthetic primer
<400> 140
   aattcaattt caactcctgt tgat 24
<210> 141
   <211> 18
   <212> DNA
   <213> Artificial Sequence
<220>
   <223> Description of Artificial Sequence: Synthetic primer
<400> 141
   ttttatcgct gtggcctt 18
<210> 142
   <211> 19
   <212> DNA
   <213> Artificial Sequence
<220>
   <223> Description of Artificial Sequence: Synthetic primer
<400> 142
   ctttacaaca aggccagac 19
<210> 143
   <211> 22
   <212> DNA
   <213> Artificial Sequence
<220>
   <223> Description of Artificial Sequence: Synthetic primer
<400> 143
   cgatcactaa atatgtgatg cc 22
<210> 144
   <211> 33
   <212> DNA
   <213> Artificial Sequence
<220>
   <223> Description of Artificial Sequence: Synthetic primer
<400> 144
   gttgttcttt gttatttttt ctattagttt att 33
<210> 145
   <211> 28
   <212> DNA
   <213> Artificial Sequence
<220>
   <223> Description of Artificial Sequence: Synthetic primer
<400> 145
   cttcgtgctt tacatatttt aaaacatt 28
<210> 146
   <211> 22
   <212> DNA
   <213> Artificial Sequence
<220>
   <223> Description of Artificial Sequence: Synthetic primer
<400> 146
   gagaagtcct attaagatcg ct 22
<210> 147
   <211> 22
   <212> DNA
   <213> Artificial Sequence
<220>
   <223> Description of Artificial Sequence: Synthetic primer
<400> 147
   ctgtgaaaac tcccgattta tc 22
<210> 148
   <211> 23
   <212> DNA
   <213> Artificial Sequence
<220>
   <223> Description of Artificial Sequence: Synthetic primer
<400> 148
   catttgttat tggatgaaat gcg 23
<210> 149
   <211> 17
   <212> DNA
   <213> Artificial Sequence
<220>
   <223> Description of Artificial Sequence: Synthetic primer
<400> 149
   gcttccaacc caaattg 17
<210> 150
   <211> 17
   <212> DNA
   <213> Artificial Sequence
<220>
   <223> Description of Artificial Sequence: Synthetic primer
<400> 150
   cggttccgta agttcct 17
<210> 151
   <211> 24
   <212> DNA
   <213> Artificial Sequence
<220>
   <223> Description of Artificial Sequence: Synthetic primer
<400> 151
   tctgcttctc aaaatgtaag aaca 24
<210> 152
   <211> 24
   <212> DNA
   <213> Artificial Sequence
<220>
   <223> Description of Artificial Sequence: Synthetic primer
<400> 152
   taaccaaatg cacatgttat caaa 24
<210> 153
   <211> 20
   <212> DNA
   <213> Artificial Sequence
<220>
   <223> Description of Artificial Sequence: Synthetic primer
<400> 153
   ttgctgatca agctcaatat 20
<210> 154
   <211> 22
   <212> DNA
   <213> Artificial Sequence
<220>
   <223> Description of Artificial Sequence: Synthetic primer
<400> 154
   gcaacttaca gacgaaatta at 22
<210> 155
   <211> 24
   <212> DNA
   <213> Artificial Sequence
<220>
   <223> Description of Artificial Sequence: Synthetic primer
<400> 155
   agacagaggt tctatacaaa ttga 24
<210> 156
   <211> 21
   <212> DNA
   <213> Artificial Sequence
<220>
   <223> Description of Artificial Sequence: Synthetic primer
<400> 156
   aggtaacggc acatattcag a 21
<210> 157
   <211> 23
   <212> DNA
   <213> Artificial Sequence
<220>
   <223> Description of Artificial Sequence: Synthetic primer
<400> 157
   taagaatgaa ggaattggca gtt 23
<210> 158
   <211> 29
   <212> DNA
   <213> Artificial Sequence
<220>
   <223> Description of Artificial Sequence: Synthetic primer
<400> 158
   aatttaaatg aagtagaaaa agtcttagt 29
<210> 159
   <211> 27
   <212> DNA
   <213> Artificial Sequence
<220>
   <223> Description of Artificial Sequence: Synthetic primer
<400> 159
   ggctattaat tttattcaga caacaga 27
<210> 160
   <211> 22
   <212> DNA
   <213> Artificial Sequence
<220>
   <223> Description of Artificial Sequence: Synthetic primer
<400> 160
   ttgtcacaag cttctagaaa ta 22
<210> 161
   <211> 25
   <212> DNA
   <213> Artificial Sequence
<220>
   <223> Description of Artificial Sequence: Synthetic primer
<400> 161
   tttctggtaa gattaatgct caaat 25
<210> 162
   <211> 20
   <212> DNA
   <213> Artificial Sequence
<220>
   <223> Description of Artificial Sequence: Synthetic primer
<400> 162
   gagcttctga tgatgctgct 20
<210> 163
   <211> 21
   <212> DNA
   <213> Artificial Sequence
<220>
   <223> Description of Artificial Sequence: Synthetic primer
<400> 163
   gaaaagcttt ctagtgggta c 21
<210> 164
   <211> 24
   <212> DNA
   <213> Artificial Sequence
<220>
   <223> Description of Artificial Sequence: Synthetic primer
<400> 164
   cattaacgct gctaatctta gtaa 24
<210> 165
   <211> 23
   <212> DNA
   <213> Artificial Sequence
<220>
   <223> Description of Artificial Sequence: Synthetic primer
<400> 165
   catcagctat taatgcttca aga 23
<210> 166
   <211> 28
   <212> DNA
   <213> Artificial Sequence
<220>
   <223> Description of Artificial Sequence: Synthetic primer
<400> 166
   catggaggaa tgatatatga ttatcatg 28
<210> 167
   <211> 33
   <212> DNA
   <213> Artificial Sequence
<220>
   <223> Description of Artificial Sequence: Synthetic primer
<400> 167
   ttttttttta atttttgtgc tattcttttt aac 33
<210> 168
   <211> 35
   <212> DNA
   <213> Artificial Sequence
<220>
   <223> Description of Artificial Sequence: Synthetic primer
<400> 168
   taataataat tatttttaat gctattgcta tttgc 35
<210> 169
   <211> 28
   <212> DNA
   <213> Artificial Sequence
<220>
   <223> Description of Artificial Sequence: Synthetic primer
<400> 169
   attaaaggct tttgatttta atcaaaga 28
<210> 170
   <211> 21
   <212> DNA
   <213> Artificial Sequence
<220>
   <223> Description of Artificial Sequence: Synthetic primer
<400> 170
   ttaagcgcat gaaagatcaa g 21
<210> 171
   <211> 21
   <212> DNA
   <213> Artificial Sequence
<220>
   <223> Description of Artificial Sequence: Synthetic primer
<400> 171
   gtggaaggtg aacttaatac c 21
<210> 172
   <211> 30
   <212> DNA
   <213> Artificial Sequence
<220>
   <223> Description of Artificial Sequence: Synthetic primer
<400> 172
   gattataaaa agaagtacga agatagagag 30
<210> 173
   <211> 33
   <212> DNA
   <213> Artificial Sequence
<220>
   <223> Description of Artificial Sequence: Synthetic primer
<400> 173
   ttattttttt gattaaaaat tttcaagtcg taa 33
<210> 174
   <211> 21
   <212> DNA
   <213> Artificial Sequence
<220>
   <223> Description of Artificial Sequence: Synthetic primer
<400> 174
   gcttccggag gagttattta t 21
<210> 175
   <211> 18
   <212> DNA
   <213> Artificial Sequence
<220>
   <223> Description of Artificial Sequence: Synthetic primer
<400> 175
   taggagattg tctgtcgc 18
<210> 176
   <211> 22
   <212> DNA
   <213> Artificial Sequence
<220>
   <223> Description of Artificial Sequence: Synthetic primer
<400> 176
   gcaacattag ctgcataaat at 22
<210> 177
   <211> 18
   <212> DNA
   <213> Artificial Sequence
<220>
   <223> Description of Artificial Sequence: Synthetic primer
<400> 177
   tccctcacca gagaaaag 18
<210> 178
   <211> 19
   <212> DNA
   <213> Artificial Sequence
<220>
   <223> Description of Artificial Sequence: Synthetic primer
<400> 178
   acaccctctt gaaccggtg 19
<210> 179
   <211> 20
   <212> DNA
   <213> Artificial Sequence
<220>
   <223> Description of Artificial Sequence: Synthetic primer
<400> 179
   tgagaaggtg ctgtagcagg 20
<210> 180
   <211> 27
   <212> DNA
   <213> Artificial Sequence
<220>
   <223> Description of Artificial Sequence: Synthetic primer
<400> 180
   ttgtaacatt aacaggagaa ttaactc 27
<210> 181
   <211> 24
   <212> DNA
   <213> Artificial Sequence
<220>
   <223> Description of Artificial Sequence: Synthetic primer
<400> 181
   ttagcaagtg atgtattagc atca 24
<210> 182
   <211> 27
   <212> DNA
   <213> Artificial Sequence
<220>
   <223> Description of Artificial Sequence: Synthetic primer
<400> 182
   tgatcactta tcattctaat agcattt 27
<210> 183
   <211> 22
   <212> DNA
   <213> Artificial Sequence
<220>
   <223> Description of Artificial Sequence: Synthetic primer
<400> 183
   ctattttgga aagcacctaa at 22
<210> 184
   <211> 27
   <212> DNA
   <213> Artificial Sequence
<220>
   <223> Description of Artificial Sequence: Synthetic primer
<400> 184
   gcatactcag tactattctt tatagat 27
<210> 185
   <211> 24
   <212> DNA
   <213> Artificial Sequence
<220>
   <223> Description of Artificial Sequence: Synthetic primer
<400> 185
   tgagcataag atgcttttag attt 24
<210> 186
   <211> 22
   <212> DNA
   <213> Artificial Sequence
<220>
   <223> Description of Artificial Sequence: Synthetic primer
<400> 186
   tctgtcattg tagcatcttt ta 22
<210> 187
   <211> 28
   <212> DNA
   <213> Artificial Sequence
<220>
   <223> Description of Artificial Sequence: Synthetic primer
<400> 187
   ttaaaatact attagttgtt gctgctac 28
<210> 188
   <211> 18
   <212> DNA
   <213> Artificial Sequence
<220>
   <223> Description of Artificial Sequence: Synthetic primer
<400> 188
   attagcctgc gcaatcat 18
<210> 189
   <211> 21
   <212> DNA
   <213> Artificial Sequence
<220>
   <223> Description of Artificial Sequence: Synthetic primer
<400> 189
   gcaatgacaa aacatattgg g 21
<210> 190
   <211> 34
   <212> DNA
   <213> Artificial Sequence
<220>
   <223> Description of Artificial Sequence: Synthetic primer
<400> 190
   ttaatacaat ttataccaat taaactagaa tttt 34
<210> 191
   <211> 30
   <212> DNA
   <213> Artificial Sequence
<220>
   <223> Description of Artificial Sequence: Synthetic primer
<400> 191
   ataaaaaaac aaaagatcct ttaaaggatc 30
<210> 192
   <211> 35
   <212> DNA
   <213> Artificial Sequence
<220>
   <223> Description of Artificial Sequence: Synthetic primer
<400> 192
   ataaattata ctaaaattat taaatttttg ccgat 35
<210> 193
   <211> 22
   <212> DNA
   <213> Artificial Sequence
<220>
   <223> Description of Artificial Sequence: Synthetic primer
<400> 193
   gcctgcatta tgctttataa ca 22
<210> 194
   <211> 19
   <212> DNA
   <213> Artificial Sequence
<220>
   <223> Description of Artificial Sequence: Synthetic primer
<400> 194
   cctactcaaa gcaaactcc 19
<210> 195
   <211> 34
   <212> DNA
   <213> Artificial Sequence
<220>
   <223> Description of Artificial Sequence: Synthetic primer
<400> 195
   cgaaaatact ttataacaat ctttaatttt aaca 34
<210> 196
   <211> 24
   <212> DNA
   <213> Artificial Sequence
<220>
   <223> Description of Artificial Sequence: Synthetic primer
<400> 196
   tcgacttatc tgctttttgt taac 24
<210> 197
   <211> 23
   <212> DNA
   <213> Artificial Sequence
<220>
   <223> Description of Artificial Sequence: Synthetic primer
<400> 197
   ctatctttgc catcttcata gtc 23
<210> 198
   <211> 29
   <212> DNA
   <213> Artificial Sequence
<220>
   <223> Description of Artificial Sequence: Synthetic primer
<400> 198
   gcaataaaaa tagaagattc tttgtagat 29
<210> 199
   <211> 34
   <212> DNA
   <213> Artificial Sequence
<220>
   <223> Description of Artificial Sequence: Synthetic primer
<400> 199
   taaaatttca ttttcataaa catcaagatt aata 34
<210> 200
   <211> 14
   <212> DNA
   <213> Artificial Sequence
<220>
   <223> Description of Artificial Sequence: Synthetic primer
<400> 200
   gcccgacata ccca 14
<210> 201
   <211> 31
   <212> DNA
   <213> Artificial Sequence
<220>
   <223> Description of Artificial Sequence: Synthetic primer
<400> 201
   ttaatgaaaa agaatacatt aagtgcaata t 31
<210> 202
   <211> 29
   <212> DNA
   <213> Artificial Sequence
<220>
   <223> Description of Artificial Sequence: Synthetic primer
<400> 202
   ctaataattc ataaataaaa aggaggcac 29
<210> 203
   <211> 32
   <212> DNA
   <213> Artificial Sequence
<220>
   <223> Description of Artificial Sequence: Synthetic primer
<400> 203
   ttttcaaata aaaaattgaa aaacaaaatt gt 32
<210> 204
   <211> 34
   <212> DNA
   <213> Artificial Sequence
<220>
   <223> Description of Artificial Sequence: Synthetic primer
<400> 204
   aatatttatt caagatattg aagaatttga aaaa 34
<210> 205
   <211> 35
   <212> DNA
   <213> Artificial Sequence
<220>
   <223> Description of Artificial Sequence: Synthetic primer
<400> 205
   tttaaaatca aattaagaca atatttttca aattc 35
<210> 206
   <211> 22
   <212> DNA
   <213> Artificial Sequence
<220>
   <223> Description of Artificial Sequence: Synthetic primer
<400> 206
   agcatatttg gctttgctta tg 22
<210> 207
   <211> 38
   <212> DNA
   <213> Artificial Sequence
<220>
   <223> Description of Artificial Sequence: Synthetic primer
<400> 207
   aaattaaaac tttttttatt aaagtatact tcatttaa 38
<210> 208
   <211> 25
   <212> DNA
   <213> Artificial Sequence
<220>
   <223> Description of Artificial Sequence: Synthetic primer
<400> 208
   gcctgagtat tcattatata agtcc 25
<210> 209
   <211> 28
   <212> DNA
   <213> Artificial Sequence
<220>
   <223> Description of Artificial Sequence: Synthetic primer
<400> 209
   tatattggga tccaaaatct aatacaag 28
<210> 210
   <211> 28
   <212> DNA
   <213> Artificial Sequence
<220>
   <223> Description of Artificial Sequence: Synthetic primer
<400> 210
   caatttctct aattcttctt gcaattag 28
<210> 211
   <211> 31
   <212> DNA
   <213> Artificial Sequence
<220>
   <223> Description of Artificial Sequence: Synthetic primer
<400> 211
   ggagtatagt aaggtattac ttttgtataa a 31
<210> 212
   <211> 32
   <212> DNA
   <213> Artificial Sequence
<220>
   <223> Description of Artificial Sequence: Synthetic primer
<400> 212
   ttcctgagat attcatattt ttaatttctt tt 32
<210> 213
   <211> 19
   <212> DNA
   <213> Artificial Sequence
<220>
   <223> Description of Artificial Sequence: Synthetic primer
<400> 213
   gcaggacttc cacttagta 19
<210> 214
   <211> 24
   <212> DNA
   <213> Artificial Sequence
<220>
   <223> Description of Artificial Sequence: Synthetic primer
<400> 214
   ggtaggagct tcttttgaat aaac 24
<210> 215
   <211> 34
   <212> DNA
   <213> Artificial Sequence
<220>
   <223> Description of Artificial Sequence: Synthetic primer
<400> 215
   caaaataggt attttcaaat taaaaatttc cata 34
<210> 216
   <211> 31
   <212> DNA
   <213> Artificial Sequence
<220>
   <223> Description of Artificial Sequence: Synthetic primer
<400> 216
   aatttaacaa ttatttgcat tccataacat a 31
<210> 217
   <211> 24
   <212> DNA
   <213> Artificial Sequence
<220>
   <223> Description of Artificial Sequence: Synthetic primer
<400> 217
   gcttagagtc tttagatact aggc 24
<210> 218
   <211> 22
   <212> DNA
   <213> Artificial Sequence
<220>
   <223> Description of Artificial Sequence: Synthetic primer
<400> 218
   aaagatttca gagctcccat at 22
<210> 219
   <211> 29
   <212> DNA
   <213> Artificial Sequence
<220>
   <223> Description of Artificial Sequence: Synthetic primer
<400> 219
   ttctgaaaat aaaagagatt tttcatctc 29
<210> 220
   <211> 28
   <212> DNA
   <213> Artificial Sequence
<220>
   <223> Description of Artificial Sequence: Synthetic primer
<400> 220
   tgactcatga taatttgaaa tttgtttg 28
<210> 221
   <211> 29
   <212> DNA
   <213> Artificial Sequence
<220>
   <223> Description of Artificial Sequence: Synthetic primer
<400> 221
   aaattatcag gaattttttc aatactgtc 29
<210> 222
   <211> 29
   <212> DNA
   <213> Artificial Sequence
<220>
   <223> Description of Artificial Sequence: Synthetic primer
<400> 222
   gcaatacaat tttttgtaaa agctaattg 29
<210> 223
   <211> 27
   <212> DNA
   <213> Artificial Sequence
<220>
   <223> Description of Artificial Sequence: Synthetic primer
<400> 223
   ccgtaaattt tttgagtttc atttgat 27
<210> 224
   <211> 22
   <212> DNA
   <213> Artificial Sequence
<220>
   <223> Description of Artificial Sequence: Synthetic primer
<400> 224
   agttacttct ggatggaatt gt 22
<210> 225
   <211> 30
   <212> DNA
   <213> Artificial Sequence
<220>
   <223> Description of Artificial Sequence: Synthetic primer
<400> 225
   aatttttaat tatttgatca ccaaattcag 30
<210> 226
   <211> 20
   <212> DNA
   <213> Artificial Sequence
<220>
   <223> Description of Artificial Sequence: Synthetic primer
<400> 226
   accgcattag aatccgtaat 20
<210> 227
   <211> 19
   <212> DNA
   <213> Artificial Sequence
<220>
   <223> Description of Artificial Sequence: Synthetic primer
<400> 227
   acctctttca cagcaagtt 19
<210> 228
   <211> 21
   <212> DNA
   <213> Artificial Sequence
<220>
   <223> Description of Artificial Sequence: Synthetic primer
<400> 228
   catctataga tgacagcaac g 21
<210> 229
   <211> 21
   <212> DNA
   <213> Artificial Sequence
<220>
   <223> Description of Artificial Sequence: Synthetic primer
<400> 229
   ttaccaatag ctttagcagc a 21
<210> 230
   <211> 25
   <212> DNA
   <213> Artificial Sequence
<220>
   <223> Description of Artificial Sequence: Synthetic primer
<400> 230
   gtatccaaac cattattttg gtgta 25
<210> 231
   <211> 25
   <212> DNA
   <213> Artificial Sequence
<220>
   <223> Description of Artificial Sequence: Synthetic primer
<400> 231
   gctaacaatg atccattgtg attat 25
<210> 232
   <211> 24
   <212> DNA
   <213> Artificial Sequence
<220>
   <223> Description of Artificial Sequence: Synthetic primer
<400> 232
   tattagggtt gatattgcat aagc 24
<210> 233
   <211> 28
   <212> DNA
   <213> Artificial Sequence
<220>
   <223> Description of Artificial Sequence: Synthetic primer
<400> 233
   cttcattttt caatccatct aatttttg 28
<210> 234
   <211> 21
   <212> DNA
   <213> Artificial Sequence
<220>
   <223> Description of Artificial Sequence: Synthetic primer
<400> 234
   ttagccgcat caattttttc c 21
<210> 235
   <211> 32
   <212> DNA
   <213> Artificial Sequence
<220>
   <223> Description of Artificial Sequence: Synthetic primer
<400> 235
   tcttttaatt tattagtaaa tgtttcagaa ca 32
<210> 236
   <211> 22
   <212> DNA
   <213> Artificial Sequence
<220>
   <223> Description of Artificial Sequence: Synthetic primer
<400> 236
   cttctttacc aagatctgtg tg 22
<210> 237
   <211> 19
   <212> DNA
   <213> Artificial Sequence
<220>
   <223> Description of Artificial Sequence: Synthetic primer
<400> 237
   cttttgcatc agcatcagt 19
<210> 238
   <211> 31
   <212> DNA
   <213> Artificial Sequence
<220>
   <223> Description of Artificial Sequence: Synthetic primer
<400> 238
   tttagtttta gtaccatttg tttttaaaat g 31
<210> 239
   <211> 28
   <212> DNA
   <213> Artificial Sequence
<220>
   <223> Description of Artificial Sequence: Synthetic primer
<400> 239
   gattcaaata attttccaag ttcttcag 28
<210> 240
   <211> 19
   <212> DNA
   <213> Artificial Sequence
<220>
   <223> Description of Artificial Sequence: Synthetic primer
<400> 240
   ctgcttttga caagacctc 19
<210> 241
   <211> 24
   <212> DNA
   <213> Artificial Sequence
<220>
   <223> Description of Artificial Sequence: Synthetic primer
<400> 241
   tttaactgaa ttagcaagca tctc 24
<210> 242
   <211> 15
   <212> DNA
   <213> Artificial Sequence
<220>
   <223> Description of Artificial Sequence: Synthetic primer
<400> 242
   ccacaacagg gcttg 15
<210> 243
   <211> 27
   <212> DNA
   <213> Artificial Sequence
<220>
   <223> Description of Artificial Sequence: Synthetic primer
<400> 243
   gatcttaatt aaggtttttt tggactt 27
<210> 244
   <211> 33
   <212> DNA
   <213> Artificial Sequence
<220>
   <223> Description of Artificial Sequence: Synthetic primer
<400> 244
   ccagttactt ttttaaaaca aattaatctt ata 33
<210> 245
   <211> 29
   <212> DNA
   <213> Artificial Sequence
<220>
   <223> Description of Artificial Sequence: Synthetic primer
<400> 245
   agaaatcttt cttgacttat attgacttt 29
<210> 246
   <211> 39
   <212> DNA
   <213> Artificial Sequence
<220>
   <223> Description of Artificial Sequence: Synthetic primer
<400> 246
   gaattttaag aaattttttg agaaaataaa aaaataaaa 39
<210> 247
   <211> 27
   <212> DNA
   <213> Artificial Sequence
<220>
   <223> Description of Artificial Sequence: Synthetic primer
<400> 247
   tattctttaa gagaagagct taaagtt 27
<210> 248
   <211> 30
   <212> DNA
   <213> Artificial Sequence
<220>
   <223> Description of Artificial Sequence: Synthetic primer
<400> 248
   aaattcaatt tattaacggc ttttgtaata 30
<210> 249
   <211> 27
   <212> DNA
   <213> Artificial Sequence
<220>
   <223> Description of Artificial Sequence: Synthetic primer
<400> 249
   tctagcaccc aattttgttt atattta 27
<210> 250
   <211> 29
   <212> DNA
   <213> Artificial Sequence
<220>
   <223> Description of Artificial Sequence: Synthetic primer
<400> 250
   gtttaagcct acttaaagtc tttaaaatc 29
<210> 251
   <211> 20
   <212> DNA
   <213> Artificial Sequence
<220>
   <223> Description of Artificial Sequence: Synthetic primer
<400> 251
   cccacactct ctctttcaaa 20
<210> 252
   <211> 23
   <212> DNA
   <213> Artificial Sequence
<220>
   <223> Description of Artificial Sequence: Synthetic primer
<400> 252
   gatattaacc ggcatttaac ctt 23
<210> 253
   <211> 26
   <212> DNA
   <213> Artificial Sequence
<220>
   <223> Description of Artificial Sequence: Synthetic primer
<400> 253
   tctagcttac aatcccattt ataaga 26
<210> 254
   <211> 31
   <212> DNA
   <213> Artificial Sequence
<220>
   <223> Description of Artificial Sequence: Synthetic primer
<400> 254
   ccttcaaatt ttaattttcc tctaaaagtt a 31
<210> 255
   <211> 26
   <212> DNA
   <213> Artificial Sequence
<220>
   <223> Description of Artificial Sequence: Synthetic primer
<400> 255
   ccttcaaaag aagaatcaag atacaa 26
<210> 256
   <211> 20
   <212> DNA
   <213> Artificial Sequence
<220>
   <223> Description of Artificial Sequence: Synthetic primer
<400> 256
   cacaccccct tttgaagata 20
<210> 257
   <211> 27
   <212> DNA
   <213> Artificial Sequence
<220>
   <223> Description of Artificial Sequence: Synthetic primer
<400> 257
   gtaataacct tactattctt gccaata 27
<210> 258
   <211> 30
   <212> DNA
   <213> Artificial Sequence
<220>
   <223> Description of Artificial Sequence: Synthetic primer
<400> 258
   ttctactatt aatgtatcac aaattaccac 30
<210> 259
   <211> 20
   <212> DNA
   <213> Artificial Sequence
<220>
   <223> Description of Artificial Sequence: Synthetic primer
<400> 259
   gcatttacat tgcccttcaa 20
<210> 260
   <211> 23
   <212> DNA
   <213> Artificial Sequence
<220>
   <223> Description of Artificial Sequence: Synthetic primer
<400> 260
   caaccgctgt ttaaataaac ctt 23
<210> 261
   <211> 30
   <212> DNA
   <213> Artificial Sequence
<220>
   <223> Description of Artificial Sequence: Synthetic primer
<400> 261
   aatatttttt ttgtttttac atccccatat 30
<210> 262
   <211> 30
   <212> DNA
   <213> Artificial Sequence
<220>
   <223> Description of Artificial Sequence: Synthetic primer
<400> 262
   cacacttacc atcaaaaatt atattatcat 30
<210> 263
   <211> 33
   <212> DNA
   <213> Artificial Sequence
<220>
   <223> Description of Artificial Sequence: Synthetic primer
<400> 263
   aagaaaataa atctacaatt tcattagact tta 33
<210> 264
   <211> 26
   <212> DNA
   <213> Artificial Sequence
<220>
   <223> Description of Artificial Sequence: Synthetic primer
<400> 264
   caaagtatct tttatttgtg aaacgg 26
<210> 265
   <211> 34
   <212> DNA
   <213> Artificial Sequence
<220>
   <223> Description of Artificial Sequence: Synthetic primer
<400> 265
   tctacttatt attaattaat aaaaaacact gacc 34
<210> 266
   <211> 33
   <212> DNA
   <213> Artificial Sequence
<220>
   <223> Description of Artificial Sequence: Synthetic primer
<400> 266
   ctctacgaat taaattttta agaaaggatt tta 33
<210> 267
   <211> 26
   <212> DNA
   <213> Artificial Sequence
<220>
   <223> Description of Artificial Sequence: Synthetic primer
<400> 267
   atcaaatcca ccattttttt tatcca 26
<210> 268
   <211> 18
   <212> DNA
   <213> Artificial Sequence
<220>
   <223> Description of Artificial Sequence: Synthetic primer
<400> 268
   ccaaccgcct tatttcac 18
<210> 269
   <211> 33
   <212> DNA
   <213> Artificial Sequence
<220>
   <223> Description of Artificial Sequence: Synthetic primer
<400> 269
   ttttcaaatt atcttcaatc ttaaactctt tag 33
<210> 270
   <211> 25
   <212> DNA
   <213> Artificial Sequence
<220>
   <223> Description of Artificial Sequence: Synthetic primer
<400> 270
   ttttagcaac aactttaacc acttt 25
<210> 271
   <211> 17
   <212> DNA
   <213> Artificial Sequence
<220>
   <223> Description of Artificial Sequence: Synthetic primer
<400> 271
   tgtcacgcta gatgcag 17
<210> 272
   <211> 21
   <212> DNA
   <213> Artificial Sequence
<220>
   <223> Description of Artificial Sequence: Synthetic primer
<400> 272
   ctttacgcca cttaaatctg c 21
<210> 273
   <211> 24
   <212> DNA
   <213> Artificial Sequence
<220>
   <223> Description of Artificial Sequence: Synthetic primer
<400> 273
   aatcagaaaa tattaccccg tttg 24
<210> 274
   <211> 31
   <212> DNA
   <213> Artificial Sequence
<220>
   <223> Description of Artificial Sequence: Synthetic primer
<400> 274
   atattatttt ctaaacctga agaaggaata t 31
<210> 275
   <211> 31
   <212> DNA
   <213> Artificial Sequence
<220>
   <223> Description of Artificial Sequence: Synthetic primer
<400> 275
   cattaaaaaa tttgatgata ttactttgct c 31
<210> 276
   <211> 27
   <212> DNA
   <213> Artificial Sequence
<220>
   <223> Description of Artificial Sequence: Synthetic primer
<400> 276
   gttttgctgt taaagtaagg aaattag 27
<210> 277
   <211> 22
   <212> DNA
   <213> Artificial Sequence
<220>
   <223> Description of Artificial Sequence: Synthetic primer
<400> 277
   gctgctagaa aaaaatctcg tt 22
<210> 278
   <211> 25
   <212> DNA
   <213> Artificial Sequence
<220>
   <223> Description of Artificial Sequence: Synthetic primer
<400> 278
   ctgctagaaa gcgaataatt cataa 25
<210> 279
   <211> 31
   <212> DNA
   <213> Artificial Sequence
<220>
   <223> Description of Artificial Sequence: Synthetic primer
<400> 279
   gaatttttta aatttgttgc aaaaaaacta g 31
<210> 280
   <211> 18
   <212> DNA
   <213> Artificial Sequence
<220>
   <223> Description of Artificial Sequence: Synthetic primer
<400> 280
   gcgggtaaga aagacgaa 18
<210> 281
   <211> 22
   <212> DNA
   <213> Artificial Sequence
<220>
   <223> Description of Artificial Sequence: Synthetic primer
<400> 281
   gaaaaacgct gtatcaacat ga 22
<210> 282
   <211> 33
   <212> DNA
   <213> Artificial Sequence
<220>
   <223> Description of Artificial Sequence: Synthetic primer
<400> 282
   attagaaatg taagtgtaaa aagtgaatta aaa 33
<210> 283
   <211> 23
   <212> DNA
   <213> Artificial Sequence
<220>
   <223> Description of Artificial Sequence: Synthetic primer
<400> 283
   cgctctcgtc aaaatttaaa aag 23
<210> 284
   <211> 21
   <212> DNA
   <213> Artificial Sequence
<220>
   <223> Description of Artificial Sequence: Synthetic primer
<400> 284
   cttgagaaaa aatgcatctg c 21
<210> 285
   <211> 38
   <212> DNA
   <213> Artificial Sequence
<220>
   <223> Description of Artificial Sequence: Synthetic primer
<400> 285
   gatatattaa agctattgtt taataatatt attaagga 38
<210> 286
   <211> 34
   <212> DNA
   <213> Artificial Sequence
<220>
   <223> Description of Artificial Sequence: Synthetic primer
<400> 286
   attaacttaa atctttgatt gactatattt gaat 34
<210> 287
   <211> 32
   <212> DNA
   <213> Artificial Sequence
<220>
   <223> Description of Artificial Sequence: Synthetic primer
<400> 287
   aggtttttga atatattaat caaaactatt gt 32
<210> 288
   <211> 31
   <212> DNA
   <213> Artificial Sequence
<220>
   <223> Description of Artificial Sequence: Synthetic primer
<400> 288
   attttgaata aaaaaatttc ttattccatg c 31
<210> 289
   <211> 26
   <212> DNA
   <213> Artificial Sequence
<220>
   <223> Description of Artificial Sequence: Synthetic primer
<400> 289
   caaagaaaat catcagacaa aaaagg 26
<210> 290
   <211> 35
   <212> DNA
   <213> Artificial Sequence
<220>
   <223> Description of Artificial Sequence: Synthetic primer
<400> 290
   gaatttgaat ttaacaataa aaattattta tgctt 35
<210> 291
   <211> 29
   <212> DNA
   <213> Artificial Sequence
<220>
   <223> Description of Artificial Sequence: Synthetic primer
<400> 291
   gaattttttg aaaaaatttt tattgccag 29
<210> 292
   <211> 30
   <212> DNA
   <213> Artificial Sequence
<220>
   <223> Description of Artificial Sequence: Synthetic primer
<400> 292
   ctgtgaaaga aaaattttta aaagtgaaat 30
<210> 293
   <211> 32
   <212> DNA
   <213> Artificial Sequence
<220>
   <223> Description of Artificial Sequence: Synthetic primer
<400> 293
   caatatagtg ttattttatg agtttaggaa ag 32
<210> 294
   <211> 21
   <212> DNA
   <213> Artificial Sequence
<220>
   <223> Description of Artificial Sequence: Synthetic primer
<400> 294
   ttttgttggg ggatttttca g 21
<210> 295
   <211> 21
   <212> DNA
   <213> Artificial Sequence
<220>
   <223> Description of Artificial Sequence: Synthetic primer
<400> 295
   ttttgttggg ggatttttca g 21
<210> 296
   <211> 42
   <212> DNA
   <213> Artificial Sequence
<220>
   <223> Description of Artificial Sequence: Synthetic primer
<400> 296
   gcaatatatt tattttttta tttatttgtt ttattgatat 42
<210> 297
   <211> 34
   <212> DNA
   <213> Artificial Sequence
<220>
   <223> Description of Artificial Sequence: Synthetic primer
<400> 297
   gtattatgat tgctttattt gtttattaca tttc 34
<210> 298
   <211> 36
   <212> DNA
   <213> Artificial Sequence
<220>
   <223> Description of Artificial Sequence: Synthetic primer
<400> 298
   gatattattt atcttgtact tatcttttta tgtttt 36
<210> 299
   <211> 23
   <212> DNA
   <213> Artificial Sequence
<220>
   <223> Description of Artificial Sequence: Synthetic primer
<400> 299
   gtcccaaaat tggaaaattt tcc 23
<210> 300
   <211> 33
   <212> DNA
   <213> Artificial Sequence
<220>
   <223> Description of Artificial Sequence: Synthetic primer
<400> 300
   tatttaaaga gcttaaaatt aagagaaaag atc 33
<210> 301
   <211> 18
   <212> DNA
   <213> Artificial Sequence
<220>
   <223> Description of Artificial Sequence: Synthetic primer
<400> 301
   cgtgaagctg caagaaaa 18
<210> 302
   <211> 23
   <212> DNA
   <213> Artificial Sequence
<220>
   <223> Description of Artificial Sequence: Synthetic primer
<400> 302
   tggaaaagca ataaaagctg ctg 23
<210> 303
   <211> 27
   <212> DNA
   <213> Artificial Sequence
<220>
   <223> Description of Artificial Sequence: Synthetic primer
<400> 303
   tgttgtatat gaacatttat tggaaat 27
<210> 304
   <211> 24
   <212> DNA
   <213> Artificial Sequence
<220>
   <223> Description of Artificial Sequence: Synthetic primer
<400> 304
   gcttggtaat tctgagataa gaaa 24
<210> 305
   <211> 23
   <212> DNA
   <213> Artificial Sequence
<220>
   <223> Description of Artificial Sequence: Synthetic primer
<400> 305
   cctcaatttg aaggtcaaac aaa 23
<210> 306
   <211> 23
   <212> DNA
   <213> Artificial Sequence
<220>
   <223> Description of Artificial Sequence: Synthetic primer
<400> 306
   attttaaaga ggggcttaca gct 23
<210> 307
   <211> 21
   <212> DNA
   <213> Artificial Sequence
<220>
   <223> Description of Artificial Sequence: Synthetic primer
<400> 307
   gccatgaatg aagcttttaa a 21
<210> 308
   <211> 25
   <212> DNA
   <213> Artificial Sequence
<220>
   <223> Description of Artificial Sequence: Synthetic primer
<400> 308
   ctcatgttat gggatttaga agtgg 25
<210> 309
   <211> 25
   <212> DNA
   <213> Artificial Sequence
<220>
   <223> Description of Artificial Sequence: Synthetic primer
<400> 309
   ctgacaacat tctttctttt gttaa 25
<210> 310
   <211> 21
   <212> DNA
   <213> Artificial Sequence
<220>
   <223> Description of Artificial Sequence: Synthetic primer
<400> 310
   tgttaatgtg gggcttaaat g 21
<210> 311
   <211> 22
   <212> DNA
   <213> Artificial Sequence
<220>
   <223> Description of Artificial Sequence: Synthetic primer
<400> 311
   gcttttcaat cagaacctta tt 22
<210> 312
   <211> 22
   <212> DNA
   <213> Artificial Sequence
<220>
   <223> Description of Artificial Sequence: Synthetic primer
<400> 312
   gagggtggga taaaatcttt tt 22
<210> 313
   <211> 28
   <212> DNA
   <213> Artificial Sequence
<220>
   <223> Description of Artificial Sequence: Synthetic primer
<400> 313
   tggtaaagaa aaatcttcaa aattttat 28
<210> 314
   <211> 30
   <212> DNA
   <213> Artificial Sequence
<220>
   <223> Description of Artificial Sequence: Synthetic primer
<400> 314
   cgataaaata tacatttcaa ttgaagataa 30
<210> 315
   <211> 20
   <212> DNA
   <213> Artificial Sequence
<220>
   <223> Description of Artificial Sequence: Synthetic primer
<400> 315
   ggcttaaaga gcttgctttt 20
<210> 316
   <211> 28
   <212> DNA
   <213> Artificial Sequence
<220>
   <223> Description of Artificial Sequence: Synthetic primer
<400> 316
   agattataat ttcgatgttc ttgaaaaa 28
<210> 317
   <211> 25
   <212> DNA
   <213> Artificial Sequence
<220>
   <223> Description of Artificial Sequence: Synthetic primer
<400> 317
   cggattctga aatttttgaa acttt 25
<210> 318
   <211> 21
   <212> DNA
   <213> Artificial Sequence
<220>
   <223> Description of Artificial Sequence: Synthetic primer
<400> 318
   ggggactaag gttacttttt t 21
<210> 319
   <211> 25
   <212> DNA
   <213> Artificial Sequence
<220>
   <223> Description of Artificial Sequence: Synthetic primer
<400> 319
   agaagttgtg ggggaatctt ctgtt 25
<210> 320
   <211> 24
   <212> DNA
   <213> Artificial Sequence
<220>
   <223> Description of Artificial Sequence: Synthetic primer
<400> 320
   ctttttcaaa aggtattccg actt 24
<210> 321
   <211> 27
   <212> DNA
   <213> Artificial Sequence
<220>
   <223> Description of Artificial Sequence: Synthetic primer
<400> 321
   ggtttatgtt aatagagatg gaaaaat 27
<210> 322
   <211> 24
   <212> DNA
   <213> Artificial Sequence
<220>
   <223> Description of Artificial Sequence: Synthetic primer
<400> 322
   ggttgtaaat gctctatctt cgtt 24
<210> 323
   <211> 25
   <212> DNA
   <213> Artificial Sequence
<220>
   <223> Description of Artificial Sequence: Synthetic primer
<400> 323
   tggtaagttt aataaaggca cgtat 25
<210> 324
   <211> 26
   <212> DNA
   <213> Artificial Sequence
<220>
   <223> Description of Artificial Sequence: Synthetic primer
<400> 324
   ccttgaactt gttttaacaa aattac 26
<210> 325
   <211> 21
   <212> DNA
   <213> Artificial Sequence
<220>
   <223> Description of Artificial Sequence: Synthetic primer
<400> 325
   accgatattc atgaagagga g 21
<210> 326
   <211> 23
   <212> DNA
   <213> Artificial Sequence
<220>
   <223> Description of Artificial Sequence: Synthetic primer
<400> 326
   tacccatttt agcacttcct cca 23
<210> 327
   <211> 20
   <212> DNA
   <213> Artificial Sequence
<220>
   <223> Description of Artificial Sequence: Synthetic primer
<400> 327
   tggcaaaatg gcctgaaaaa 20
<210> 328
   <211> 25
   <212> DNA
   <213> Artificial Sequence
<220>
   <223> Description of Artificial Sequence: Synthetic primer
<400> 328
   ttgttttctc aacattaagc atttt 25
<210> 329
   <211> 25
   <212> DNA
   <213> Artificial Sequence
<220>
   <223> Description of Artificial Sequence: Synthetic primer
<400> 329
   atcattggtg ataaccttat cttct 25
<210> 330
   <211> 18
   <212> DNA
   <213> Artificial Sequence
<220>
   <223> Description of Artificial Sequence: Synthetic primer
<400> 330
   actcctgcac caagagat 18
<210> 331
   <211> 24
   <212> DNA
   <213> Artificial Sequence
<220>
   <223> Description of Artificial Sequence: Synthetic primer
<400> 331
   atcttgtgat aacgaagttt tgta 24
<210> 332
   <211> 20
   <212> DNA
   <213> Artificial Sequence
<220>
   <223> Description of Artificial Sequence: Synthetic primer
<400> 332
   tccatcaaca tcggcatctg 20
<210> 333
   <211> 24
   <212> DNA
   <213> Artificial Sequence
<220>
   <223> Description of Artificial Sequence: Synthetic primer
<400> 333
   aaaagctaaa agcaaagttc taat 24
<210> 334
   <211> 29
   <212> DNA
   <213> Artificial Sequence
<220>
   <223> Description of Artificial Sequence: Synthetic primer
<400> 334
   atatatccat tttcaattaa atctctcat 29
<210> 335
   <211> 20
   <212> DNA
   <213> Artificial Sequence
<220>
   <223> Description of Artificial Sequence: Synthetic primer
<400> 335
   tataaagagg aggcatggct 20
<210> 336
   <211> 30
   <212> DNA
   <213> Artificial Sequence
<220>
   <223> Description of Artificial Sequence: Synthetic primer
<400> 336
   taaaaataat aaatacgatt gtcatacttt 30
<210> 337
   <211> 26
   <212> DNA
   <213> Artificial Sequence
<220>
   <223> Description of Artificial Sequence: Synthetic primer
<400> 337
   tattgcgatt tttagtttca atagaa 26
<210> 338
   <211> 23
   <212> DNA
   <213> Artificial Sequence
<220>
   <223> Description of Artificial Sequence: Synthetic primer
<400> 338
   cccaagccct ttatatctct gaa 23
<210> 339
   <211> 18
   <212> DNA
   <213> Artificial Sequence
<220>
   <223> Description of Artificial Sequence: Synthetic primer
<400> 339
   agctgcgttg gattcatc 18
<210> 340
   <211> 22
   <212> DNA
   <213> Artificial Sequence
<220>
   <223> Description of Artificial Sequence: Synthetic primer
<400> 340
   ctagcaggat ccatagttgt tt 22
<210> 341
   <211> 28
   <212> DNA
   <213> Artificial Sequence
<220>
   <223> Description of Artificial Sequence: Synthetic primer
<400> 341
   atcatctata ttcatcaatc tcattttt 28
<210> 342
   <211> 26
   <212> DNA
   <213> Artificial Sequence
<220>
   <223> Description of Artificial Sequence: Synthetic primer
<400> 342
   gagtaacaaa aattttttca gcttca 26
<210> 343
   <211> 21
   <212> DNA
   <213> Artificial Sequence
<220>
   <223> Description of Artificial Sequence: Synthetic primer
<400> 343
   tttctgggct caactaaatc t 21
<210> 344
   <211> 28
   <212> DNA
   <213> Artificial Sequence
<220>
   <223> Description of Artificial Sequence: Synthetic primer
<400> 344
   gattaattac attaagtgca ttctgttc 28
<210> 345
   <211> 21
   <212> DNA
   <213> Artificial Sequence
<220>
   <223> Description of Artificial Sequence: Synthetic primer
<400> 345
   ccattaacgc tccaattaca c 21
<210> 346
   <211> 31
   <212> DNA
   <213> Artificial Sequence
<220>
   <223> Description of Artificial Sequence: Synthetic primer
<400> 346
   tcctaacatt taatatttgt tctttatttt c 31
<210> 347
   <211> 33
   <212> DNA
   <213> Artificial Sequence
<220>
   <223> Description of Artificial Sequence: Synthetic primer
<400> 347
   gcataattta aataagaagt ttttatttca tct 33
<210> 348
   <211> 25
   <212> DNA
   <213> Artificial Sequence
<220>
   <223> Description of Artificial Sequence: Synthetic primer
<400> 348
   gaagagctct agaaacaata actga 25
<210> 349
   <211> 23
   <212> DNA
   <213> Artificial Sequence
<220>
   <223> Description of Artificial Sequence: Synthetic primer
<400> 349
   tggtttaaga ccatctctta cgt 23
<210> 350
   <211> 28
   <212> DNA
   <213> Artificial Sequence
<220>
   <223> Description of Artificial Sequence: Synthetic primer
<400> 350
   ctcatacata gaataaagta ttctcctg 28